# EUROPEAN PATENT SPECIFICATION

(11) **EP 4 202 029 B1**
(45) Date of publication and mention of the grant of the patent: **18.09.2024**
(21) Application number: 23154016.2
(22) Date of filing: 25.06.2021
(51) Int. Cl.: C12M 1/00, C12M 3/00, C12M 1/12, C12M 3/06

(54) **METHOD OF MANUFACTURE A BIOCHIP**
VERFAHREN ZUR HERSTELLUNG EINES BIOCHIPS
PROCEDE DE FABRICATION D'UNE BIOPUCE

(30) Priority: 25.06.2020 US 202063044223 P; 06.11.2020 US 202063110673 P
(43) Date of publication of application: 28.06.2023
(62) Divisional of application: 21829706.7
(73) Proprietor: DLOC Biosystems, Inc., Aley, Mount Lebanon (LB)
(72) Inventor: MALAEB, Waddah Arkan, 22178 Doha (QA)
(74) Representative: K&L Gates LLP

(56) References cited:
- EP-B1- 3 007 882
- WO-A1-2018/237132
- WO-A1-2020/073043
- WO-A2-2017/003546
- US-A1- 2001 036 674
- US-A1- 2014 011 707
- US-A1- 2018 036 727

## Description

### BACKGROUND

Developing a successful pharmaceutical drug is expensive and time-consuming. Additionally, many drugs fail in late stages of the development process, causing significant sunk costs. One contributing reason for drugs failing in late developmental stages is that pharmaceutical companies typically test drugs on cells grown on ineffective cell culture platforms that do not sufficiently replicate human tissue. For instance, typical cell culture platforms are often composed of 2D flat plates. Existing 2D flat plates, however, do not replicate the 3D structure of human organs, resulting in inaccurate results. For example, one typical microstructure of human tissue is circular duct morphology, which is the microstructure of many organs such as the breast, pancreas, liver, kidney, and vasculature. Typical 2D flat plates do not resemble the 3D microstructure of circular duct morphology.

Two fundamental aspects for the creation of a biomimetic model are preserving cell polarity through structural scaffolding and inducing cell signal exchange through co-culturing the epithelial tissue with the basement membrane and the surrounding stroma. Cell polarity preservation is important when testing on epithelial ductal tissues because polarized cells reach senescence when formed into a duct, and thus stop growing, as compared to cancerous counterparts that do not have a demarcated polarity. The cancerous cells therefore never reach senescence and keep dividing or growing, leading to uncontrollable cancer cell growth. Accordingly, cells cultured and hooked on each other forming a close duct resembles normal epithelial tissue, whereas cells cultured in 2D function similar to the cells at the duct formation stage. Growing cells in an abnormal environment therefore may make them function more as a cancerous tissue than a normal tissue.

Co-culturing epithelial cells with the surrounding stroma is important because fibrous tissue plays a vital role in the ductal cancer progression through hormonal and insulin-like growth factor (IGF) level fluctuations. Such fluctuations cause changes in the stromal cells' gene expression, leading to different extracellular matrix biomarkers, and thus disrupting the signaling cascades from and to the epithelial tissues. Typically, systems that have one of those two fundamental aspects show increased resemblance to native tissue when compared to a typical 2D culture platform or 3D gels, but still show many major differences when compared to the native tissue.

Another important aspect for the creation of a biomimetic model is the capability to test a drug's effect on the tissue microenvironment. This means that for an assembled tissue, it is important to test the effect of a modification in one tissue environment on that of the surrounding tissue. This is especially important in cancer applications, particularly due to drastic changes in the extracellular matrix (ECM) leading to an alteration of the signaling pathways in the tissue cells and the cells in the surrounding tissues. These drastic changes have been shown to play a vital role in cancer progression and metastasis.

Typical cell culture platforms are incapable of co-culturing different types of cells in spatially separated compartments, which makes it difficult to model the full microenvironment of human tissue. For example, a breast tumor microenvironment includes normal epithelial cells forming a duct as well as breast cancer cells. Both cell types are interacting with the supporting cells in the extracellular matrix. This represents the tumor microenvironment that affects the action of drugs aimed to treat breast cancer. Traditional drug discovery platforms and culture systems, however, do not recapitulate the circular structure of normal breast cells, and do not capture the effect of other cells in the tumor microenvironment on the cancer cells.

WO 2018/237132 discloses a method of manufacturing a microfluidic device comprising obtaining a base mold with at least one protruding chamber and at least one rod which spans from one edge of the mold through the chamber to the opposite edge of the mold; bonding the solidified polymer mold to a surface, inserting extracellular matrix hydrogel into the chamber and removing the at least one rod once the matrix hydrogel has polymerized.

EP 3 007 882 discloses a method for manufacturing a microfluidic device using a 3D-printing method for microfluidic channels having a cylindrical shape.

Accordingly, there is a need for cell culture platforms for testing drugs and a method of manufacturing a biochip that solve the above drawbacks.

### SUMMARY

The present disclosure provides a new and innovative method of manufacturing a biochip that acts as a cell culture platform. Cells can be inserted into the biochip and grow into 3-dimentional tissues to be used in drug testing. Inside the biochip is an ultra-thin porous plastic duct that is formed by curving membranes into a cylindrical shape. The duct can be accessed from both sides through microfluidic channels. An aim of the provided biochip is to replicate a ducal organoid microenvironment by growing the ductal epithelial or endothelial cells on the inner walls of the duct and growing the surrounding tissue formed from the cells from the outside by seeding cells and media components through a gel from the other side. The biochip may be utilized to replicate ductal tissue including, but not limited to: pancreas, renal, hepatic, breast, lung, vasculature, prostate, testicular and lymph ducts. The biochip could be used by researchers, pharmaceutical companies and clinical physicians to test their drugs and components on the organoids grown inside the chip in order to better predict their effects before testing on humans.

The present disclosure provides a method to create a scaffolding system for cells to grow into 3D interfacing ductal tissues (cylindrical epithelial/endothelial tissue surrounded by a stroma with myoepithelial tissue and/or stroma lining in between). The disclosure allows structural stability capable to support tissues of low stiffness 3D tissues.

The present disclosure encompasses several technical advantages such as, circular cross-sectional scaffold where cells, when attached to the inner surface of the duct, and getting confluent, can achieve a membrane polarity (differentiation) resembling natural tissues and allow for co-culturing of cells with the surrounding stroma. This provides for increased mimicry resulting in more accurate prediction of the in-vivo environment and versatility of the organ-on chip. Finally, the present disclosure provides the ability to achieve high throughput capabilities where hundreds and even thousands of experiments could be performed on the disclosed chips.

In another aspect of the present disclosure, which may be used in combination with any other aspect or combination of aspects listed herein, a biochip for growing ductal tissue includes at least one membrane structure, wherein the at least one membrane structure includes at least one porous membrane configured to provide a mimetic cellular environment, at least one chassis, wherein the at least one chassis includes, a channel configured to support the at least one membrane structure and at least one microfluidic channel in fluid communication with the channel supporting the at least one membrane structure and at least one cover slip, wherein the at least one chassis is configured such that an internal space is provided within the at least one chassis and capable of creating at least one channel within the at least one chassis, wherein the internal space created between the chassis provides a compartment that is internal relative to the body of the chassis but external relative to the membrane structure and wherein a plurality of openings are provided on the at least one chassis to allow fluid or air to enter or exit the internal space created between the chassis that provides an external compartment relative to the membrane structure created between the chassis.

In another aspect of the present disclosure, which may be used in combination with any other aspect or combination of aspects listed herein, the at least one membrane structure is one or more cylindrical scaffolds.

In another aspect of the present disclosure, which may be used in combination with any other aspect or combination of aspects listed herein, the at least one membrane structure is capable of being combined within the internal space within the cylindrical scaffolds of the at least one membrane structure, providing layers of porous membrane ductal scaffolds nested within each other.

The at least one membrane structure is selected from the group of synthetic polymers, organic polymers or composite material.

In another aspect of the present disclosure, which may be used in combination with any other aspect or combination of aspects listed herein, the at least one membrane structure is capable of mimicking the in-vivo tissue conditions for different or the same biological material.

In another aspect of the present disclosure, which may be used in combination with any other aspect or combination of aspects listed herein, the membrane structure is capable of providing an environment for a plurality of stromal tissue types.

In another aspect of the present disclosure, which may be used in combination with any other aspect or combination of aspects listed herein, the at least one membrane structure is capable of providing an environment for the testing of a plurality of disease models.

In another aspect of the present disclosure, which may be used in combination with any other aspect or combination of aspects listed herein, the at least one chassis contains features that hold the cylindrical ductal scaffold in position giving access to the internal and the external compartments of the ductal scaffold.

In another aspect of the present disclosure, which may be used in combination with any other aspect or combination of aspects listed herein, the biological components may be pipetted or pumped into the microfluidic channels of the biochip.

In another aspect of the present disclosure, which may be used in combination with any other aspect or combination of aspects listed herein, the biological components are the same biological components.

In another aspect of the present disclosure, which may be used in combination with any other aspect or combination of aspects listed herein, the biological components are different biological components.

In another aspect of the present disclosure, which may be used in combination with any other aspect or combination of aspects listed herein, the at least one chassis contains features forming one or more microfluidic channels giving access to the internal and the external compartments of the ductal scaffold.

In another aspect of the present disclosure, which may be used in combination with any other aspect or combination of aspects listed herein, the at least one chassis contains features forming the microfluidic channels leading to the internal and the external compartments of the ductal scaffold.

In another aspect of the present disclosure, which may be used in combination with any other aspect or combination of aspects listed herein, the at least one chassis contains microfluidic channels leading to the internal and the external compartments of the ductal scaffold are interconnected at one or more areas of the porous ductal scaffold locations.

In another aspect of the present disclosure, which may be used in combination with any other aspect or combination of aspects listed herein, the at least one chassis contains microfluidic channels leading to the inner and external compartments of the ductal scaffold are interconnected, is only separated by the walls of the porous ductal scaffold after assembly.

In another aspect of the present disclosure, which may be used in combination with any other aspect or combination of aspects listed herein, the at least one chassis contains microfluidic channels leading to the inner and external compartments of the ductal scaffold are interconnected, are only connected through the pores on the walls of the ductal scaffold after it's assembly.

In another aspect of the present disclosure, which may be used in combination with any other aspect or combination of aspects listed herein, the features of the at least one chassis forming the microfluidic channels leading to the external compartment of the ductal scaffold could be engraved in the inner layers of the at least one chassis.

In another aspect of the present disclosure, which may be used in combination with any other aspect or combination of aspects listed herein, the features of the at least one chassis forming the microfluidic channels leading to the external compartment of the ductal scaffold could be engraved on the outer surfaces of the at least one chassis and enclosed by the at least one thin coverslip creating the full channel.

In another aspect of the present disclosure, which may be used in combination with any other aspect or combination of aspects listed herein, the cylindrical duct cross-section could be circular, ellipsoidal or any other enclosed shape.

In another aspect of the present disclosure, which may be used in combination with any other aspect or combination of aspects listed herein, the duct could be porous and the pores could be of any count, shape and size.

In another aspect of the present disclosure, which may be used in combination with any other aspect or combination of aspects listed herein, some pores allow for the diffusion of biological components.

In another aspect of the present disclosure, which may be used in combination with any other aspect or combination of aspects listed herein, some pores allow for the migration of cells across the duct wall.

In another aspect of the present disclosure, which may be used in combination with any other aspect or combination of aspects listed herein, the at least one chassis include at least one inlet and at least one outlet holes.

In another aspect of the present disclosure, which may be used in combination with any other aspect or combination of aspects listed herein, the features of the at least one chassis, forming the microfluidic channels leading to the internal compartment of the ductal scaffold, could extend between the inlet and outlet holes of the channel.

In another aspect of the present disclosure, which may be used in combination with any other aspect or combination of aspects listed herein, the features of the at least one chassis, forming the microfluidic channels leading to the internal compartment of the ductal scaffold, could extend beyond the inlet and outlet holes of the channel and later be plugged post-assembly of the chip sub-components.

In another aspect of the present disclosure, which may be used in combination with any other aspect or combination of aspects listed herein, the at least one chassis is configured to be used with a microscope or imaging device.

In another aspect of the present disclosure, which may be used in combination with any other aspect or combination of aspects listed herein, the microfluidic channels allowing fluids to flow to the internal and external compartment surrounding the ductal scaffold are enclosed between the at least one chassis.

In another aspect of the present disclosure, which may be used in combination with any other aspect or combination of aspects listed herein, the microfluidic channels allowing fluids to flow to the external compartment surrounding the ductal scaffold are enclosed between the at least one chassis and the at least one coverslip glass covering the additional side of the biochip not enclosed by the at least one chassis.

In another aspect of the present disclosure, which may be used in combination with any other aspect or combination of aspects listed herein, the at least one membrane structure bonded to the at least one chassis could be surrounded with the stromal microfluidic channel void from all sides when the membrane is curved and bonded prior to assembly in an area on the ductal scaffold surface along its length.

In another aspect of the present disclosure, which may be used in combination with any other aspect or combination of aspects listed herein, the at least one chassis is made of a material that is one of brittle, transparent and low autofluorescence such as glass or polymer.

In another aspect of the present disclosure, which may be used in combination with any other aspect or combination of aspects listed herein, the at least one chassis is made of a material that is opaque.

In another aspect of the present disclosure, which may be used in combination with any other aspect or combination of aspects listed herein, the at least one chassis is configured to deform, in response to a stimulus, and encapsulated the at least one membrane structure.

In another aspect of the present disclosure, which may be used in combination with any other aspect or combination of aspects listed herein, the stimulus is one of heat, pressure, chemical exposure or radiation exposure.

In another aspect of the present disclosure, which may be used in combination with any other aspect or combination of aspects listed herein, the at least one cover slip and the at least one chassis are integrated to form a unitary body.

In another aspect of the present disclosure, which may be used in combination with any other aspect or combination of aspects listed herein, the at least one cover slip is made of a material that is transparent.

In another aspect of the present disclosure, which may be used in combination with any other aspect or combination of aspects listed herein, the biochip is capable of being connected to and interacting with a plurality of additional biochips.

In another aspect of the present disclosure, which may be used in combination with any other aspect or combination of aspects listed herein, a method of manufacturing a biochip, the method includes providing at least one chassis, wherein the at least one chassis includes, a channel configured to support the at least one membrane structure and at least one microfluidic channel in fluid communication with the channel, providing at least one porous membrane, curving at least one porous membrane into a closed loop of cylindrical cross-section wherein a round, cylindrical porous duct is formed by curving a first part of a porous membrane and a one or more additional porous membranes form a second part of the duct, and inserting the at least one porous membrane between the at least one chassis.

In another aspect of the present disclosure, which may be used in combination with any other aspect or combination of aspects listed herein, the at least one porous membrane is curved between 0° to 180° from a plane parallel to the top surface between at least one chassis to form a full duct shape and the other membranes are curved to form the rest of the full duct.

In another aspect of the present disclosure, which may be used in combination with any other aspect or combination of aspects listed herein, the at least one chassis is configured to contain features forming the microfluidic channels leading to the internal and the external compartments of the ductal scaffold.

In another aspect of the present disclosure, which may be used in combination with any other aspect or combination of aspects listed herein, the at least one membrane structure is combined within the internal space within the cylindrical scaffolds of the at least one membrane structure, providing layers of porous membrane ductal scaffolds nested within each other.

In another aspect of the present disclosure, which may be used in combination with any other aspect or combination of aspects listed herein, the at least one chassis is configured so that microfluidic channels leading to the internal and the external compartments of the ductal scaffold are interconnected at one or more areas of the porous ductal scaffold locations.

In another aspect of the present disclosure, which may be used in combination with any other aspect or combination of aspects listed herein, the at least one chassis is configured so that the microfluidic channels leading to the inner and external compartments of the ductal scaffold are interconnected, is only separated by the walls of the porous ductal scaffold after assembly.

In another aspect of the present disclosure, which may be used in combination with any other aspect or combination of aspects listed herein, the at least one chassis is configured so that the microfluidic channels leading to the inner and external compartments of the ductal scaffold are interconnected, are only connected through the pores on the walls of the ductal scaffold after assembly.

In another aspect of the present disclosure, which may be used in combination with any other aspect or combination of aspects listed herein, the features of the at least one chassis forming the microfluidic channels leading to the external compartment of the ductal scaffold could be engraved in the inner layers of the at least one chassis.

In another aspect of the present disclosure, which may be used in combination with any other aspect or combination of aspects listed herein, the features of the at least one chassis forming the microfluidic channels leading to the external compartment of the ductal scaffold could be engraved on the outer surfaces of the at least one chassis, and covered with another chassis part or at least one coverslip creating the full channel.

In another aspect of the present disclosure, which may be used in combination with any other aspect or combination of aspects listed herein, the features the at least one chassis is configured to provide at least one inlet and at least one outlet hole providing access to the channel.

In another aspect of the present disclosure, which may be used in combination with any other aspect or combination of aspects listed herein, the features of the at least one chassis forming the microfluidic channels leading to the internal compartment of the ductal scaffold could extend between the inlet and outlet holes of the channel.

In another aspect of the present disclosure, which may be used in combination with any other aspect or combination of aspects listed herein, the features of the at least one chassis forming the microfluidic channels leading to the internal compartment of the ductal scaffold could extend beyond the inlet and outlet holes of the channel and later be plugged post-assembly of the chip sub-components.

In another aspect of the present disclosure, which may be used in combination with any other aspect or combination of aspects listed herein, the cylindrical duct is formed by bonding the membranes and the at least one chassis by one of or a combination of chemical bonding, pressure bonding, or heat bonding.

In another aspect of the present disclosure, which may be used in combination with any other aspect or combination of aspects listed herein, the curved membranes forming the cylindrical ductal scaffold can be bonded prior or post to assembly in the at least one chassis.

In another aspect of the present disclosure, which may be used in combination with any other aspect or combination of aspects listed herein, the bonding method is used to melt a controlled thickness of the materials surfaces, welding the different parts together.

In another aspect of the present disclosure, which may be used in combination with any other aspect or combination of aspects listed herein, the chemical bonding contains a mixture of ethanol and chloroform.

In another aspect of the present disclosure, which may be used in combination with any other aspect or combination of aspects listed herein, the heat bonding can include surface irradiation.

In another aspect of the present disclosure, which may be used in combination with any other aspect or combination of aspects listed herein, the at least one coverslip glass forming the top and bottom layers of the biochip is bonded to the at least one chassis using the same method as the other parts or using a glass-polymeric glue.

In another aspect of the present disclosure, which may be used in combination with any other aspect or combination of aspects listed herein, the plugs closing the extremities of the microfluidic channels leading to the ductal scaffold inner compartment, are bonded or using a polymeric glue.

In another aspect of the present disclosure, which may be used in combination with any other aspect or combination of aspects listed herein, the extension of the ductal scaffold membrane are held at its extremities and tensioned to prevent any wrinkling in the membranes.

In another aspect of the present disclosure, which may be used in combination with any other aspect or combination of aspects listed herein, a round porous duct is formed by curving a flat membrane over a rod in a closed loop of cylindrical cross-section and bonding it in an area on the ductal scaffold surface along its length.

In another aspect of the present disclosure, which may be used in combination with any other aspect or combination of aspects listed herein, a round porous duct is formed by curving a flat membrane over a rod in a closed loop of cylindrical cross-section and bonding it in a flat area along the surface of the ductal scaffold that is an extension of the duct surface.

In another aspect of the present disclosure, which may be used in combination with any other aspect or combination of aspects listed herein, a round porous duct is formed by curving more than one flat membrane over a rod in a closed loop of cylindrical cross-section and bonding it in a flat area along the surface of the ductal scaffold that is extension of the duct surface.

In another aspect of the present disclosure, which may be used in combination with any other aspect or combination of aspects listed herein, forming the membranes includes a rod holding the membrane into its desired shapes is located in between the at least one chassis, with the extremities of the ductal scaffold is bonded to the chassis and surrounded by the at least one chassis.

### BRIEF DESCRIPTION OF THE DRAWINGS

Fig. 1A illustrates a perspective view of a biochip.
Fig. 1B illustrates a top view of a biochip.
Fig. 2A illustrates an exploded view of a biochip, prior to bonding of the membranes.
Fig. 2B illustrates an exploded view of a biochip with pre-bonded membranes, prior to tube formation, according to an aspect of the present disclosure.
Fig. 2C illustrates an exploded view of a biochip after tube formation.
Fig. 2D illustrates an exploded view of a biochip, utilizing additional membranes.
Fig. 2E illustrates an exploded view of a biochip, utilizing a membrane structure formed into a tube formation which is assembled in one chassis.
Fig. 2F illustrates an exploded view of a biochip, utilizing additional membranes formed into a tube formation which is an inner duct nested within another porous membrane structure.
Fig. 3A illustrates a porous membrane forming the ductal scaffold.
Fig. 3B illustrates a porous membrane forming the ductal scaffold and bonded at the membrane extremities.
Fig. 3C illustrates a porous membrane forming a hemi ductal scaffold.
Fig. 4A illustrates a cross sectional view of ductal scaffold with the membrane bonded and its extensions cut.
FIG. 4B illustrates a cross sectional view of ductal scaffold and the extensions of the membrane are tensioned.
Fig. 4C illustrates a cross sectional view of two hemi duct membranes with their extensions tensioned in-between the two chassis assembled together to form a cylindrical duct, and bonded post assembly to the chassis.
Fig. 4D illustrates a cross sectional view of biochip assembly where the ductal scaffold is round with no membrane extensions and nested within another ductal scaffold bonded post assembly to the single chassis.
Fig. 5A illustrates a top view of an assembled biochip.
Fig. 5B illustrates a perspective view of a duct and microfluidic chamber of a biochip.
Fig. 5C illustrates a side view of a duct and microfluidic chamber of a biochip.
Fig. 6A illustrates a top view of a biochip assembly.
Fig. 6B illustrates the bonding process of the biochip components where the ductal scaffold is pre-bonded at its extremities before assembling it to the chassis.
Fig. 7A illustrates a top view of a biochip assembly, where the membrane is pulled after being curved when assembled to apply tension.
Fig. 7B illustrates the bonding process of the biochip components where the ductal scaffold is bonded at its extremities after assembling it to the chassis.
Fig. 8A illustrates a top view of a biochip assembly where the two membranes are pulled after being curved each into a hemi-duct when assembled to apply tension.
Fig. 8B illustrates the bonding process of the biochip components where the ductal scaffold is made of two membranes inclined along with the chassis.
Fig. 9A illustrates a top view of a biochip assembly where the membranes are curved into fully round ducts with no membrane extensions prior to assembly to the chassis, wherein the membranes are pre-bonded in an area on the ductal scaffold surface along its length .
Fig. 9B illustrates the bonding process of the biochip components where the ductal scaffold is round with no membrane extensions.
Fig. 10A illustrates a top view of a biochip assembly where the two membranes are pulled after being curved each into a hemi-duct when assembled to apply tension.
Fig. 10B illustrates the bonding process of the biochip components where the ductal scaffold is made of two membranes aligned along with the chassis, pre-tensioned when sandwiched, and bonded post assembly to the chassis.
Fig. 11A illustrates a top view of a biochip assembly where the two membranes are along its length with the membrane bonded and its extensions cut when assembled in only one chassis.
Fig. 11B illustrates the bonding process of the biochip components where the membrane is curved into a 180 degrees rotation, bonded in an area on the ductal scaffold surface along its length and the ductal scaffold extensions are cut prior to assembly in only one chassis .
Fig. 12A illustrates a top view of a biochip assembly where the ductal scaffold is round with no membrane extensions and nested within another ductal scaffold, where each is pre-bonded in an area on the ductal scaffold surface along its length.
Fig. 12B illustrates the bonding process of the biochip components where the ductal scaffold is round with no membrane extensions and nested within another ductal scaffold, where each is pre-bonded in an area on the ductal scaffold surface along its length.
Fig. 13A, 13B and 13C illustrates an additional embodiments of the biochip.
Fig. 14 illustrates a circular duct morphology tissue microstructure on the porous membrane of the cylindrical duct of the biochip.
Fig. 15A illustrates a cross-sectional view of the ductal tissue model where disease-free normal epithelial or endothelial cells are grown attached to the inner walls of the internal compartment of the porous ductal scaffold.
Fig. 15B illustrates a tumor tissue disease model where disease-free normal epithelial or endothelial cells are mixed with cancerous cells and inserted at the same time in the internal ductal scaffold compartment.
Fig. 15C illustrates a tumor tissue disease model where disease-free normal cells are grown in the internal ductal scaffold compartment prior to inserting previously grown solid-tumor clusters.
Fig. 15D illustrates a stromal or parenchymal based disease model.
Fig. 16 illustrates the high throughput 96-well plate format for the biochip.

### DETAILED DESCRIPTION

The present disclosure provides a method of manufacturing a biochip according to claim 1. The biochip additionally enables more predictive in vitro testing of drugs than typical culture platforms by more accurately replicating the in vivo microenvironment as compared to typical culture platforms.

The presently disclosed biochip allows cells to grow and form 3D cylindrical channels that replicate tissue in the human body. Cylindrical ducts are embedded within the biochip. The cylindrical ducts may be constructed from porous and flexible membranes, such as membranes that are typically used for 2D cell culture (e.g., in trans-well membranes). The membranes are reshaped to form the cylindrical ducts. For instance, a membrane may be rolled over a rod, then bonded to form the cylindrical duct. In some aspects, the membrane may be bonded into the cylindrical form post to assembly with the chip. For example, they may be bonded by a combination of chemical bonding (e.g., mixed ethanol and chloroform) with pressure and heat bonding.

The cylindrical ducts influence cells to adhere, grow and reorganize themselves to form 3D cylindrical channels that resemble their shape in the human body. When cells attach to the inner surface of the duct and merge together, membrane polarity or differentiation can be achieved, which resembles natural tissues. The cylindrical ducts may have a variety of diameters, for instance, from as small as 25 micrometers to greater than 2 millimeters. The range of possible diameters enables a biochip that replicates the ductal microstructure of a wide range of organs.

Each cylindrical duct is interfaced with at least one external compartment (e.g., cylindrical porous duct having internal and external compartments). For instance, a duct may be interfaced with the external compartments via microchannels. This configuration enables multi-cell culture to be made. For example, breast ductal cells can be grown along the inner wall of the cylindrical duct, and cells from the tumor microenvironment (such as fibroblasts and immune cells) can be cultured inside a gel in the external compartments to form 3D cells which are in direct contact with the outer wall of the cylindrical duct. Biomolecules or cells can pass through the pores of the cylindrical duct to achieve cross-talk between the cells inside the external compartment and the cells inside the duct. In some instances, flow can be passed inside the cylindrical ducts in order to supply cells with growth media and bioactive drugs. In other instances, sample cells or the biomolecules secreted by them may be passed inside the cylindrical ducts for bioanalysis purposes.

The biochip may include multiple ducts and multiple surrounding channels. In at least one aspect of the present disclosure, the biochip may be configured in a 96-well plate design with multiple parallel ducts so that it can be used for high throughput drug screening purposes. In such an aspect, the system has the dimensions of a standard 96-well plate and, in some instances, can be used with a pump connected to the inlet and outlet port. In other instances, fluid can be pipetted manually into the inlet port and it will fill the entire duct with the help of capillary forces.

The biochip may be constructed of a material that is optimized for cell culture purposes. In some aspects, the biochip material is transparent, which is suitable for fluorescent microscopy analysis. In some aspects, the biochip material is hydrophilic, which helps circumvent the problems of polydimethylsiloxane (PDMS), a hydrophobic material widely used in many other cell culture platforms. The provided biochip may be manufactured via existing scalable manufacturing methods such as molding and bonding.

As used herein, "about," "approximately" and "substantially" are understood to refer to numbers in a range of numerals, for example the range of -10% to + 10% of the referenced number, preferably -5% to +5% of the referenced number, more preferably -1% to + 1% of the referenced number, most preferably -0.1% to +0.1% of the referenced number.

Furthermore, all numerical ranges herein should be understood to include all integers, whole or fractions, within the range. Moreover, these numerical ranges should be construed as providing support for a claim directed to any number or subset of numbers in that range. For example, a disclosure of from 1 to 10 should be construed as supporting a range of from 1 to 8, from 3 to 7, from 1 to 9, from 3.6 to 4.6, from 3.5 to 9.9, and so forth.

As used herein and in the appended claims, the singular form of a word includes the plural, unless the context clearly dictates otherwise. Thus, the references "a," "an" and "the" are generally inclusive of the plurals of the respective terms. For example, reference to "an organ" or "a duct" includes a plurality of such "organs" or "ducts." The term "and/or" used in the context of "X and/or Y" should be interpreted as "X," or "Y," or "X and Y."

### ASSEMBLING THE BIOCHIP

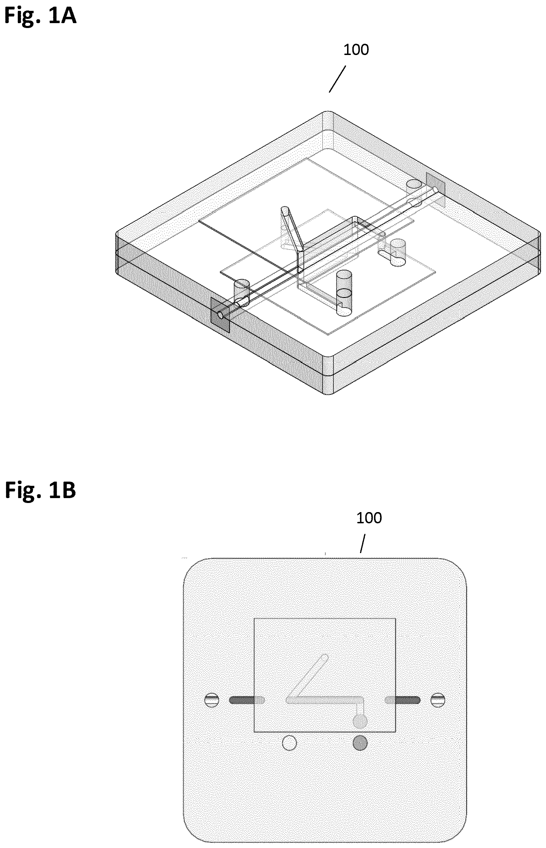

Fig. 1A and 1B illustrate a perspective view and a top view, respectively, of an assembled biochip 100, according to an aspect of the present disclosure.

In an example, the biochip 100 may be able to be supported by and used in conjunction with a microscope. For example, the biochip may be able to be placed on or held by a microscope to allow for the contents of the biochip to be viewed by the microscope. In an additional example, the biochip 100 may be compatible with an imaging device to view the processes occurring within biochip 100.

In an example, the biochip 100 may be compose of materials or components that are applicable for use with microscope. For example, the materials or components may be one of transparent, thin, provides minimal blurriness or low autofluorescence.

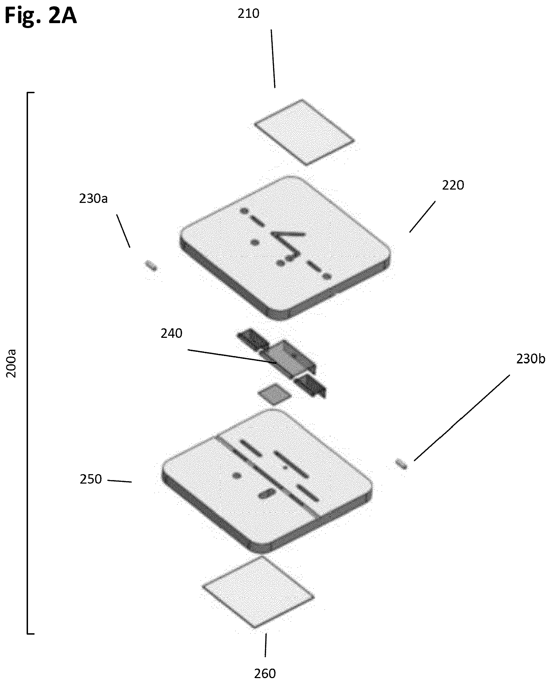

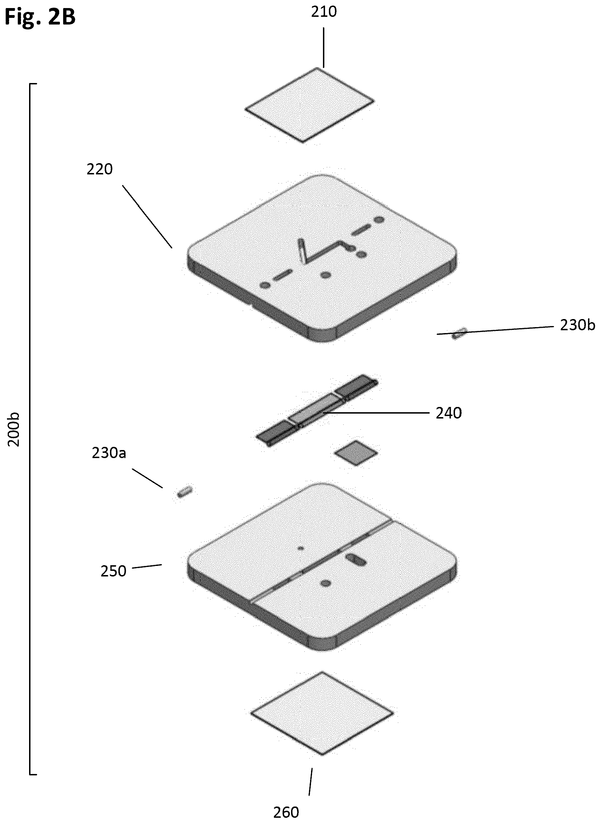

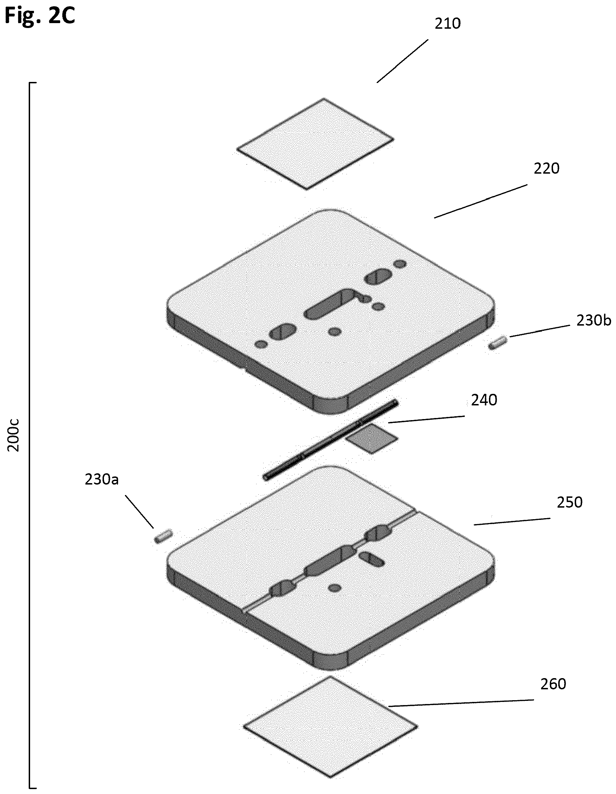

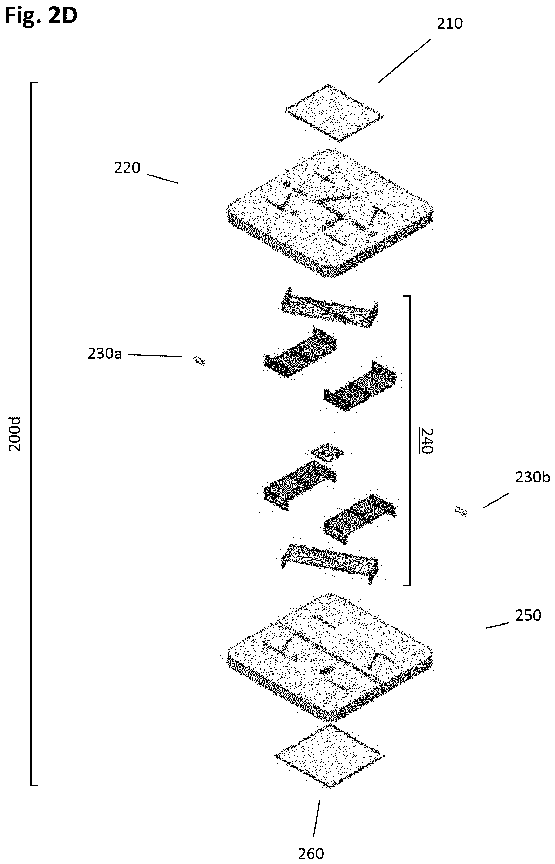

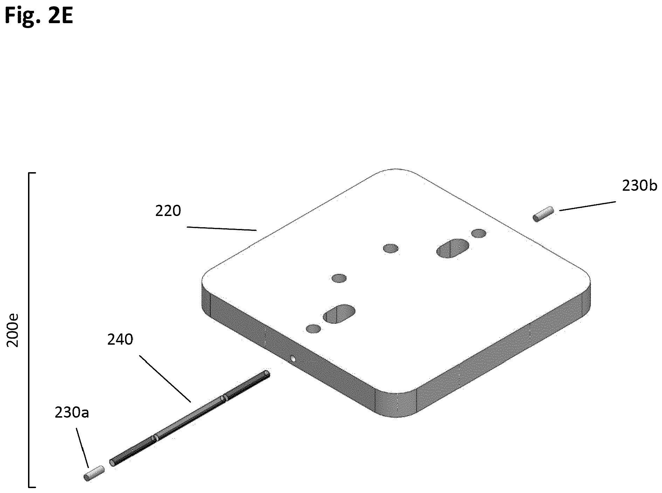

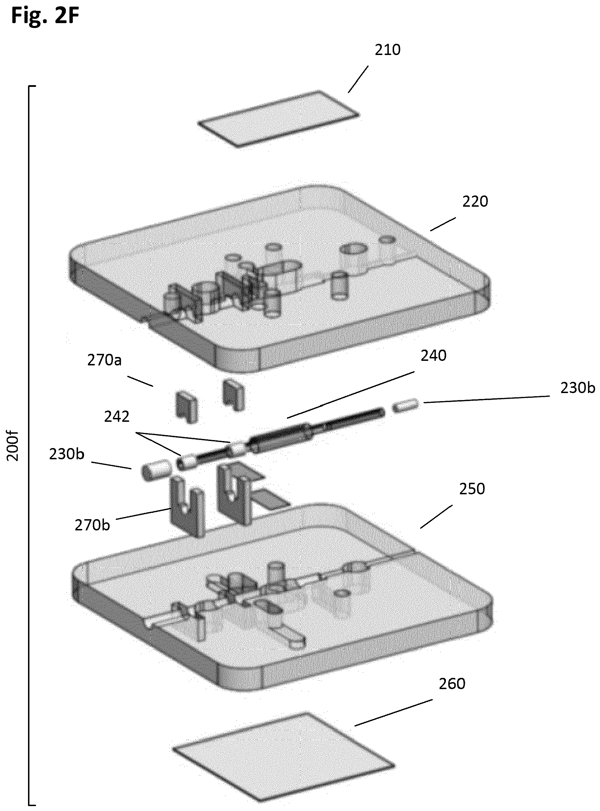

Fig. 2A, 2B, 2C, 2D, 2E and 2F illustrate the biochip 100 at through the progression of stages of assembling biochip 100. The biochip 100 is referred to as biochip 200a-200d as assembly occurs.

Referring to Fig. 2A an exploded view of a biochip 200a, illustrates an example configuration of the biochip 200a, according to an aspect of the present disclosure. In an example, the biochip 200a includes upper cover glass 210 on the uppermost portion of the biochip 200a to cover at least part of the top surface of the upper chassis 220 of the biochip 200a. The upper cover glass 210 may be a thin coverslip glass made of a material that is brittle, transparent and has low autofluorescence such as glass or polymer. The upper chassis 220 and the lower chassis 250 contain features such that when placed in direct contact with each other, an internal space is formed to accommodate porous membranes. These features may be the result of engraving in the inner layers of the chassis. In another example, upper chassis 220 and the lower chassis 250 contains features forming the microfluidic channels leading to the external compartment of the ductal scaffold and could be engraved on the outer surfaces of the chassis 250, and covered with another chassis 220 part or a thin coverslip creating the full channel on the lower chassis to accommodate the porous membrane structure 240.

The upper chassis 220 and the lower chassis 250 contain features that hold the cylindrical ductal scaffold in position giving access to the internal and the external compartments of the ductal scaffold. In another example, upper chassis 220 and the lower chassis 250 contains features forming one or more microfluidic channels giving access to the internal and the external compartments of the ductal scaffold. In another example, upper chassis 220 and the lower chassis 250 contains features microfluidic channels leading to the internal and the external compartments of the ductal scaffold are interconnected at one or more areas of the porous membrane structure 240 locations. In another example, upper chassis 220 and the lower chassis 250 contains features forming the microfluidic channels leading to the internal compartment of the ductal scaffold formed by the porous membrane structure 240 could extend between the inlet and outlet holes of the channel. The chassis 220 and 250 forming the microfluidic channels leading to the internal compartment of the ductal scaffold could extend beyond the inlet and outlet holes of the channel and later be plugged using plugs 230a and 230b post-assembly of the biochip 200a. A bottom cover glass 260, similar to the upper cover glass 210 may be provided to cover the bottom most portion of the lower chassis 250. In an example, coverslip glass 210 and 260 forming the top & bottom layers of the chip is bonded to the chassis 220 and 250 using a glass-polymeric glue.

Still referring to Fig. 2A, the porous membrane structure 240 has not been bonded yet. In an example, the porous membrane structure 240 includes two hydrophobic ultrathin porous members for filtering, a hydrophilic ultrathin porous membrane is used to form a cylindrical duct and a flat hydrophobic ultrathin porous membrane is utilized as a stroma filter.

Referring to Fig. 2B, an exploded view of a biochip 200b, which includes the same components as biochip 200a is illustrated. However here, the porous membranes forming the porous membrane structure 240 are bonded and formed into a ductal scaffold. In an example, the porous membrane structure 240 is formed by curving porous membranes into a 180° rotation, forming a cylindrical structure and bonding the access material of the membranes that were not curved into the cylindrical structure. For example, two pre-bonded cylindrical hydrophobic ultrathin porous membranes are used for filtering. Pre-bonded cylindrical hydrophilic ultrathin porous membrane are curved into a cylindrical duct Also, flat hydrophobic ultrathin porous membrane may be used as a stroma filter. These components may combine to form the porous membrane structure 240.

In an example, a round porous duct is formed by curving a single flat membrane or a plurality of flat membranes over a rod in a closed loop of cylindrical cross-section and bonding it in a flat area along the surface of the ductal scaffold that is an extension of the duct surface.

Referring to Fig. 2C, an exploded view of a biochip 200c, which includes the same components as biochip 200a is illustrated. However here, the porous membranes forming the porous membrane structure 240 have been formed into a tube formation, according to an aspect of the present disclosure. In an example, two pre-bonded cylindrical hydrophobic ultrathin porous membranes are utilized for filtering. Pre-bonded cylindrical hydrophilic ultrathin porous membranes are utilized to form the duct scaffolding. Also, flat hydrophobic ultrathin porous membranes are utilized as a stroma filter. The porous membranes are bonded in an area on the ductal scaffold surface along its length with the membrane bonded and its extensions cut prior to its assembly to the chassis 250.

Referring to Fig. 2D, an exploded view of a biochip 200d, which includes the same components as biochip 200a is illustrated. However here, additional types of porous membranes are utilized according to an additional aspect of the present disclosure. For example, two hydrophilic ultrathin porous membranes may be used to form the porous membrane structure 240 which allow for duct tissue growth area to form are utilized. Additionally, flat hydrophobic ultrathin porous membranes are utilized as a stroma air filter may also be included in porous membrane structure 240. Also, four hydrophobic ultrathin porous membranes may also be included in the porous membrane structure 240 as an air filter. In an example, two membranes forming the ductal scaffold are curved into a half-duct rotation and the extensions of the membranes are tensioned through different holes in the chassis 220 and 250 and bonded post assembly to the chassis 220 and 250.

Referring to Fig. 2E, in an example, biochip 200e, includes the porous membrane structure 240 having been formed into a tube formation which is assembled in a single chassis 220 that includes the features of both chassis (220 and 250) and coverslips (210 and 260). In an example, all individual components found in biochips 200a-200d, are integrated into a unitary single chassis 220. In an example, two pre-bonded cylindrical hydrophobic ultrathin porous membranes are utilized for filtering. Pre-bonded cylindrical hydrophilic ultrathin porous membranes are utilized to form the duct scaffoldings within each other. The porous membranes are bonded in an area on the ductal scaffold surface along its length with the membrane bonded and its extensions cut prior to its assembly to the chassis 220. The chassis 220 forming the microfluidic channels leading to the internal compartment of the ductal scaffold could extend beyond the inlet and outlet holes of the channel and later be plugged using plugs 230a and 230b post-assembly of the biochip 200e.

Referring to Fig. 2F, in an example, biochip 200f includes, chassis 220 and 250 which form the microfluidic channels leading to the internal compartment of the ductal scaffold. The inlet and outlet holes of the channel and later be plugged using plugs 230a and 230b post-assembly of the biochip 200f. In an example, a bottom cover glass 260, similar to the upper cover glass 210 may be provided to cover the bottom most portion of the lower chassis 250. In an example, coverslip glass 210 and 260 forming the top & bottom layers of the chip is bonded to the chassis 220 and 250 using a glass-polymeric glue. In an example, the porous membranes forming the porous membrane structure 240 have been formed into a tube formation which is an inner duct nested within another porous membrane structure that has been formed into a tube formation which is the intermediate duct. Two pre-bonded cylindrical hydrophobic ultrathin porous membranes are utilized for filtering. Pre-bonded cylindrical hydrophilic ultrathin porous membranes are utilized to form the duct scaffoldings within each other. Two plastic ring-shaped structures 242 are located on one of the hydrophilic porous ducts where the surrounding chassis channel has a larger diameter. Also, two flat hydrophobic ultrathin porous membranes are utilized as a stroma filter. The porous membranes are bonded in an area on the ductal scaffold surface along its length with the membrane bonded and its extensions cut prior to its assembly to the chassis 250. The chassis 220 and 250 forming the microfluidic channels leading to the internal compartment of the intermediate ductal scaffold could extend across the rings surrounding one of the hydrophobic ducts and later be plugged using puzzle-like plugs 270a and 270b from above and below getting in contact and surrounding the plastic rings post-assembly of the biochip 200a. The chassis 220 and 250, forming the microfluidic channels leading to the internal compartment of the ductal scaffold could extend beyond the inlet and outlet holes of the channel and later be plugged using plugs 230a and 230b post-assembly of the biochip 200f.

In an example, the porous membrane structure 240 may be bonded to the chassis 220 and 250 through one of chemical bonding, pressure bonding, and heat bonding. In an example, a bonding method is used to melt a controlled thickness of the materials surfaces, welding the different parts together. For example, the chemical bonding may contain a mixture of ethanol and chloroform. In another example, the heat bonding can include surface irradiation. In an example, the ductal scaffold having extension of the duct surface, when bonded post assembly to the chassis, is held at its extremities and tensioned to prevent any wrinkling in the membranes.

In an example, the porous membrane structure may be a cylindrical scaffold such that a geometry is provided that is designed to preserve the cell polarity through the differentiation markers and junction proteins on the membranes of the cells.

In an example, the porous membrane structure may have the surface properties that could be hydrophilic in some regions to support fluids, cells and biological materials access through the pores.

In an example, the porous membrane structure may have the surface properties that could be hydrophobic in some regions, to support air access through the pores, functioning as a gas access region to the tissues inside the duct.

In an example, the porous membrane structure may have the surface properties that could be hydrophobic in some regions, to support air access through the pores, functioning as an air bubble trap, removing the encapsulating air from the duct.

In an example, the porous membrane structure may have the surface properties that could be structural, mechanical and surface properties of the material constituting the ductal scaffold allow to support biological materials with various viscosities and stiffnesses.

In an example, the porous membrane structure may support the flow of fluids at high physiological flow rates and pressures.

In an example, the porous membrane structure may support inserted tissue biopsies, gels and biological materials.

In an example, ductal epithelial and endothelial tissues grown inside the duct and the tissue takes the shape of the cylindrical scaffold, preserving the cell polarity through the differentiation markers and junction proteins on the membranes of the cells.

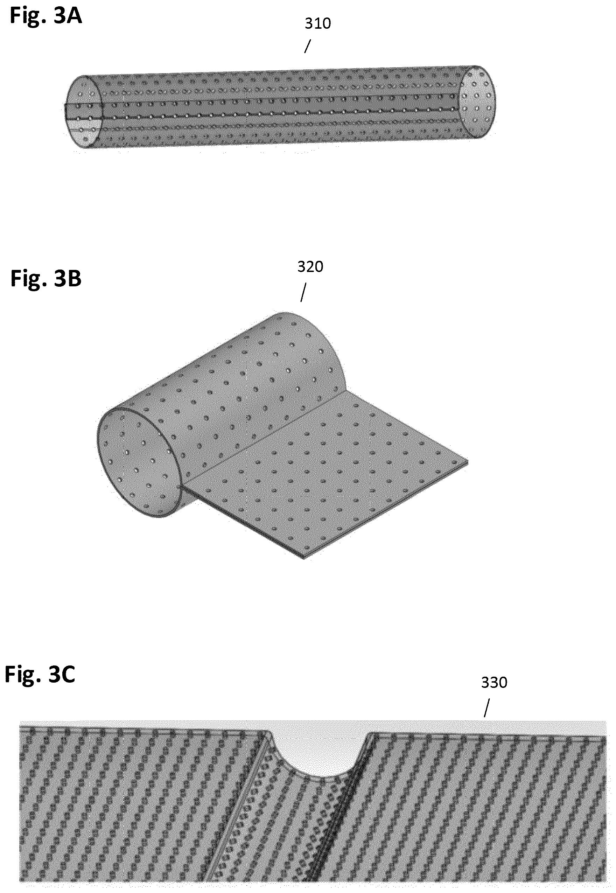

Fig. 3A, 3B and 3C illustrate various configures of porous membranes according to an aspect of the present disclosure. Fig. 3A illustrates a porous membrane 310 forming the ductal scaffold of the biochip 200d where the membrane is curved into a 180 degrees rotation, bonded in an area on the ductal scaffold surface along its length with the membrane bonded and its extensions cut prior to its assembly to the chassis, according to an aspect of the present disclosure. Fig. 3B illustrates a porous membrane 320 forming the ductal scaffold of the biochip 100, where the membrane is curved 180 degrees, and bonded at the membrane extremities, according to an aspect of the present disclosure. Fig. 3C illustrates a porous 330 membrane forming a hemi ductal scaffold of the biochip 100, that when bonded to another one at the membrane extremities, forms a cylindrical duct, according to an aspect of the present disclosure.

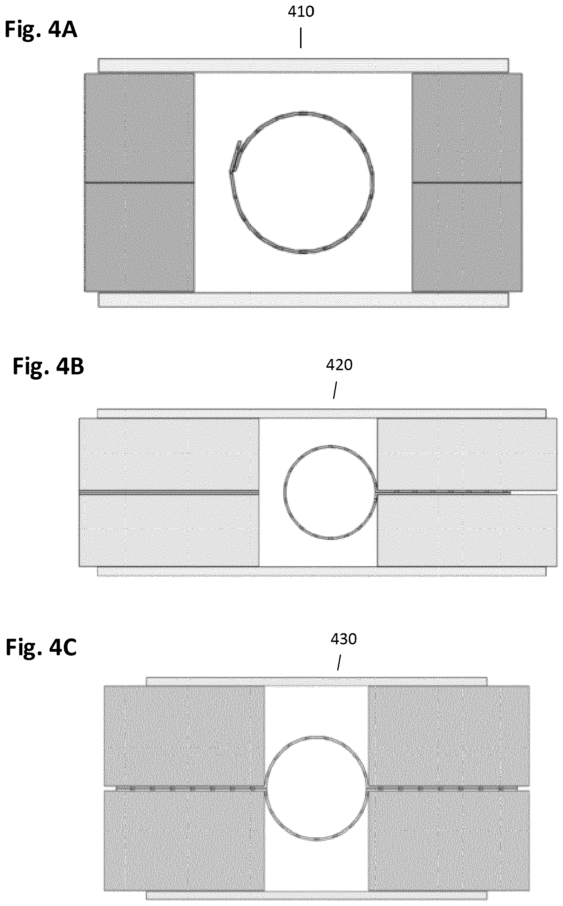

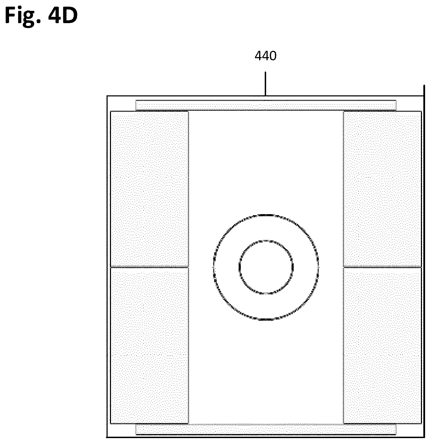

Fig. 4A, 4B, 4C and 4D illustrate cross sectional views of various configurations of assembled biochips according to an aspect of the present disclosure. Fig. 4A illustrates a cross sectional view of ductal scaffold 410 where the membrane is curved into a 180 degrees rotation, bonded in an area on the ductal scaffold 410 surface along its length with the membrane bonded and its extensions cut prior to its assembly to the chassis, according to an aspect of the present disclosure. FIG. 4B illustrates a cross sectional view of ductal scaffold 420 where the membrane is curved into a 180 degrees rotation, and the extensions of the membrane are tensioned from one side in-between the two chassis, and bonded together post assembly to the chassis, according to an aspect of the present disclosure. Fig. 4C illustrates a cross sectional view of two hemi duct membranes 430 with their extensions tensioned in-between the two chassis assembled together to form a cylindrical duct, and bonded post assembly to the chassis according to an aspect of the present disclosure. 4D illustrates a cross sectional view of biochip 440 assembly where the ductal scaffold is round with no membrane extensions and nested within another ductal scaffold bonded post assembly to the single chassis according to an aspect of the present disclosure.

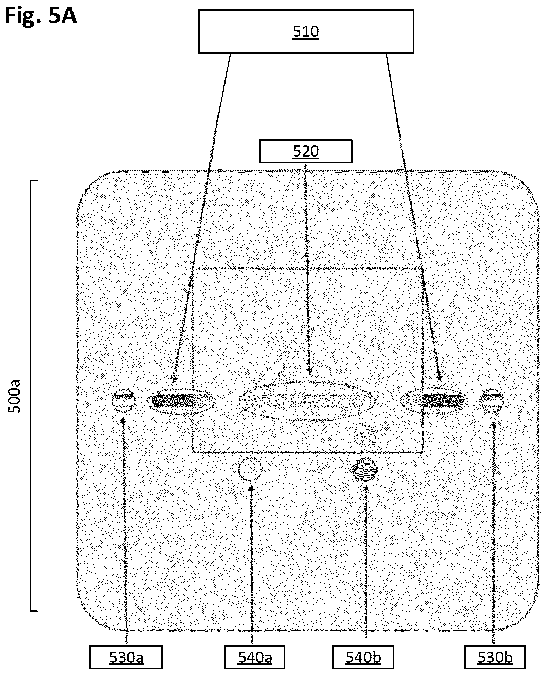

Fig. 5A illustrates a top view of a biochip 500a according to an aspect of the present disclosure. In an example, the biochip 500a may include an air filtering area 510 which may allow for air bubbles to be released out of the biochip 500a. In some examples, air filtering area 510 may also allow air to enter the biochip 500 which allows the cells being cultured to have access to air. The biochip 500a may also include a duct surrounded by a stroma 520. In some examples, the biochip 500a may include a duct inlet 530a and outlet 530b and a stroma channel inlet 540a and outlet 540b.

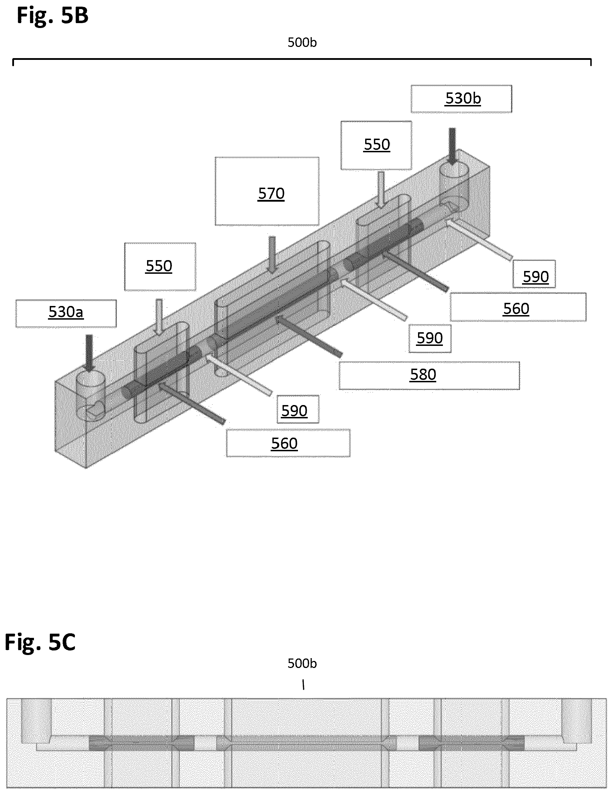

Fig. 5B illustrates a perspective view of a duct and microfluidic chamber of a biochip 500b, according to an aspect of the present disclosure. In an example, the biochip 500b may include an inlet hole 530a and an outlet hole 530b. Additionally, biochip 500b may include at least one air access area 550, at least one air bubble trap 560, a stromal microfluidic chamber 570, and a porous duct interface 580. The biochip 500b may also include multiple segments of duct surrounded with plastic 590. Fig. 5C illustrates a side view of a duct and microfluidic chamber of a biochip 500b.

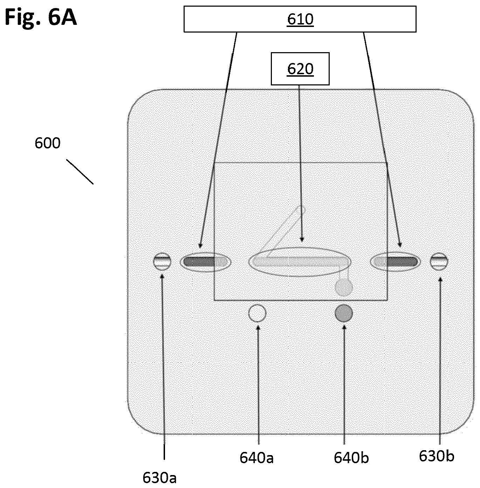

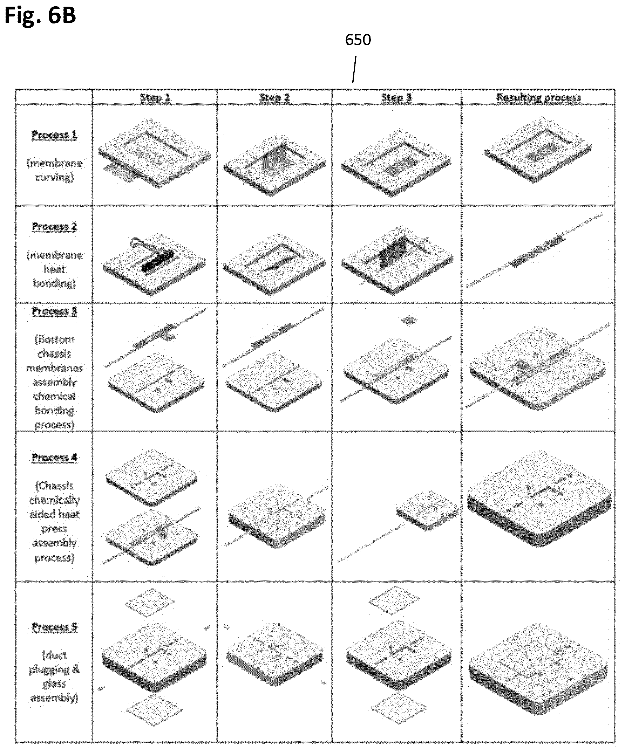

Figs. 6A, 7A, 8A, 9A and 10A illustrate a top view of the biochip 100 throughout multiple bonding methods according to an aspect of the present disclosure. Figs. 6B, 7B, 8B, 9B and 10B illustrate the steps and processes to execute the multiple bonding methods according to an aspect of the present disclosure.

### METHOD 1: Prebonding Membranes with Extensions:

Fig. 6A illustrates a top view of the biochip 100 configured according to a method where prior to bonding, porous membranes are curved in cylindrical manner, approximately in a 180° orientation. In an example, the biochip 100 configured according to this method may include, air filtering area 610, a duct surrounded by stroma 620, a duct inlet 630a, a duct outlet 630b, a stroma channel inlet 640a and a stroma channel outlet 640b.

Fig. 6B illustrates the steps and processes, according to method 650, to execute the bonding process of the biochip components where the ductal scaffold is pre-bonded at its extremities before assembling it to the chassis. For example, the method includes a curving process of the hydrophobic and hydrophilic membranes over a rod and bonding it at its extremities using heat bonding. The method may also include the assembly process of the membranes on the chassis. This may require taking the rod having the membranes bonded to it, locating it on the chassis, locating the hydrophobic flat stromal membrane in position, sandwiching everything between the two chassis, then binding them using chemical aided heat-press bonding. Finally, the method may involve the rod removal process, plugging the extremities of the ductal channel beyond the inlet and outlet holes, and assembling the coverslip glasses on both surfaces of the biochip 100.

### METHOD 2: Post-Bonding Membranes with Extensions and Perpendicular Tension:

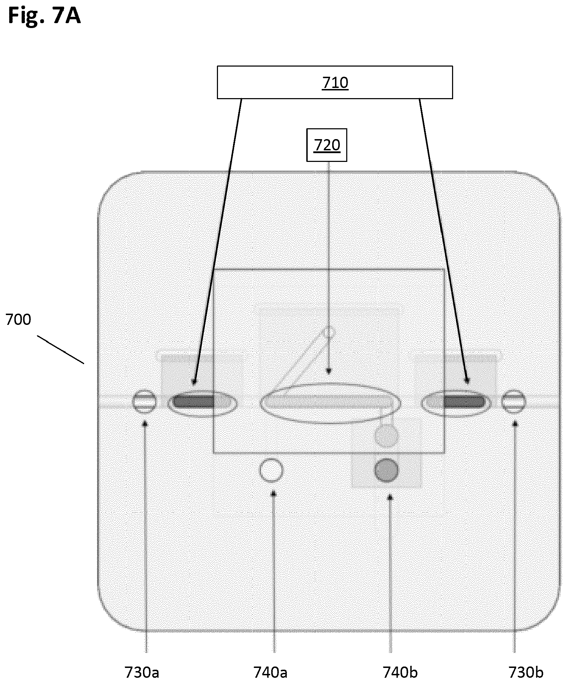

Fig. 7A illustrates a top view of the biochip 100 configured according to a method where a membrane is pulled after being curved a full 180° rotation when assembled to apply tension. In an example, the biochip 100 configured according to this method may include, air filtering area 710, a duct surrounded by stroma 720, a duct inlet 730a, a duct outlet 730b, a stroma channel inlet 740a and a stroma channel outlet 740b.

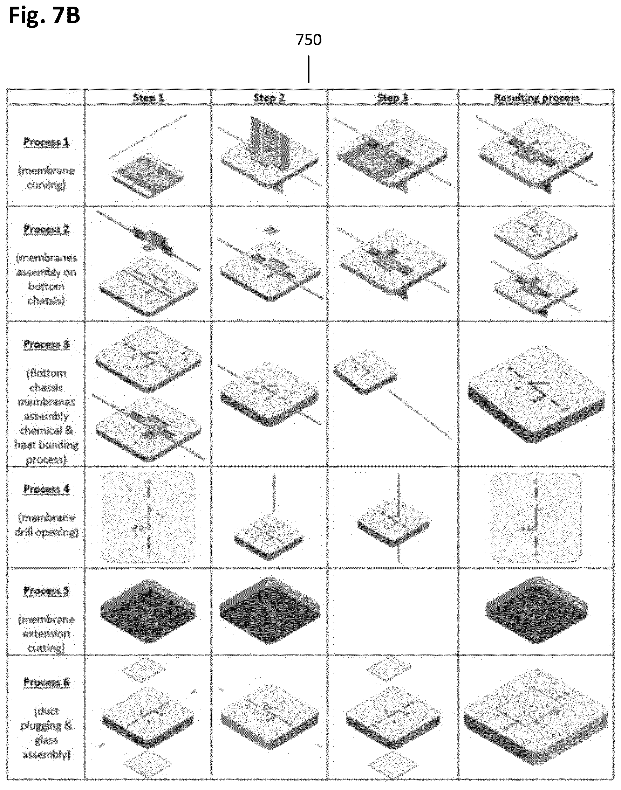

Fig. 7B illustrate the steps and processes, according to method 750, to execute the bonding process of the biochip 100 components where the ductal scaffold is bonded at its extremities after assembling it to the chassis. In an example, the method may include the curving process of the hydrophobic and hydrophilic membranes over a rod placed on one of the chassis and pulling its extremities from holes parallel to the ductal scaffold. This method may locating the hydrophobic flat stromal membrane in position, compiling the membrane layers between the two chassis, then binding them using chemical aided heat-press bonding. Finally, the method may include the rod removal process, cutting the membrane extremities, plugging the extremities of the ductal channel beyond the inlet and outlet holes, and assembling the coverslip glasses on both surfaces of the biochip 100.

### Method 3: Post-Bonding Membranes with Extensions and Inclined Tension:

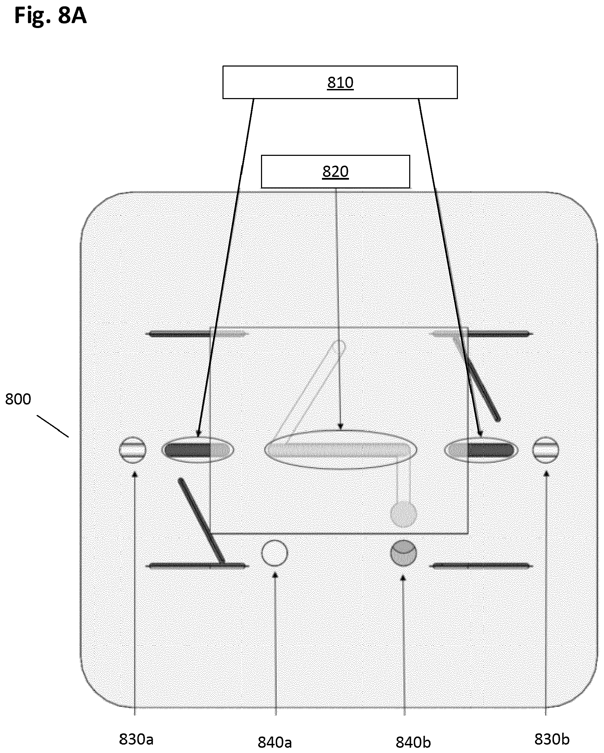

Fig. 8A illustrates a top view of the biochip 100 configured according to a method where two membranes are pulled after being curved into a hemi-duct when assembled to apply tension. In an example, the biochip 100 configured according to this method may include, air filtering area 810, a duct surrounded by stroma 820, a duct inlet 830a, a duct outlet 830b, a stroma channel inlet 840a and a stroma channel outlet 840b.

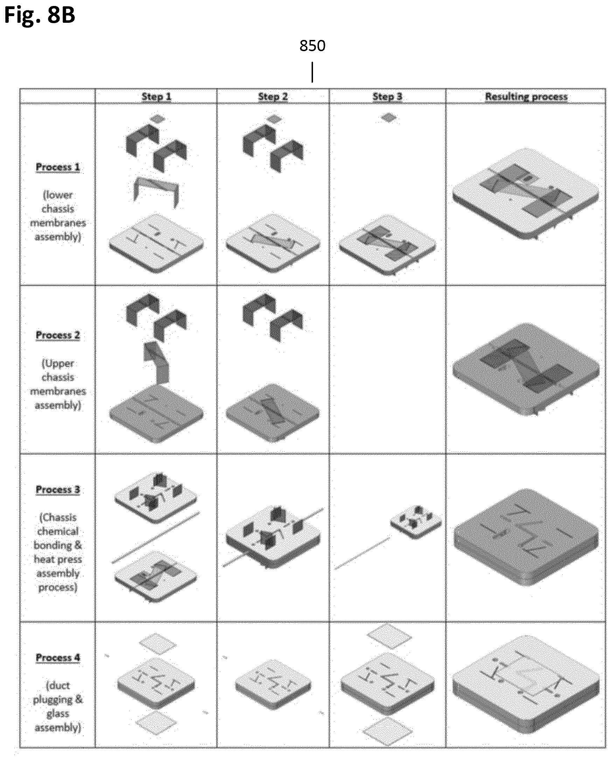

Fig. 8B illustrates the steps and processes, according to method 850, to execute the bonding process of the biochip 100 components where the ductal scaffold is made of two membranes inclined along with the chassis, pre-tensioned when sandwiched, and bonded post assembly to the chassis. In an example, the methods includes a curving process of the hydrophobic and hydrophilic membranes over a rod placed on one of the chassis and pulling its extremities from holes inclined along the ductal scaffold. The method may also include locating the hydrophobic flat stromal membrane in position, sandwiching everything between the two chassis, then binding them using chemical aided heat-press bonding. Finally, the method may include a rod removal process, cutting the membrane extremities, plugging the extremities of the ductal channel beyond the inlet and outlet holes, and assembling the coverslip glasses on both surfaces of the chip, according to an aspect of the present disclosure.

### Method 4: Pre-Bonding Membranes Fully Rounded without Extensions:

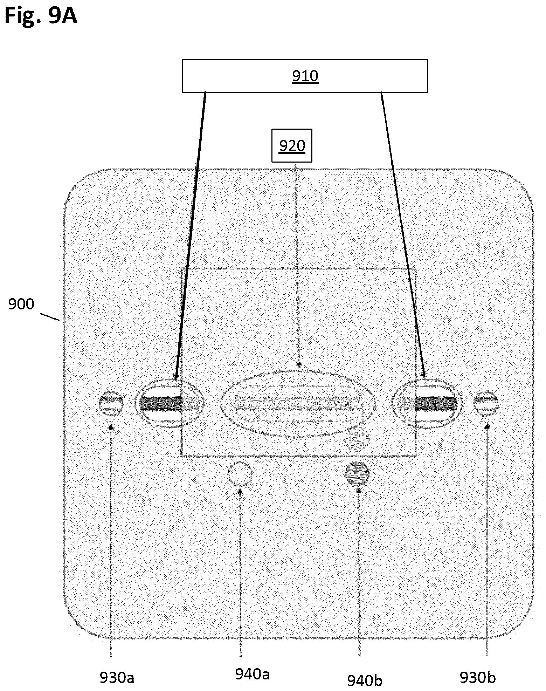

Fig. 9A illustrates a top view of the biochip 100 configured according to a method where the ductal scaffold is round with no membrane extensions, which is pre-bonded in an area on the ductal scaffold surface along its length before assembling it to the chassis. In an example, the biochip 100 configured according to this method may include, air filtering area 910, a duct surrounded by stroma 920, a duct inlet 930a, a duct outlet 930b, a stroma channel inlet 940a and a stroma channel outlet 940b.

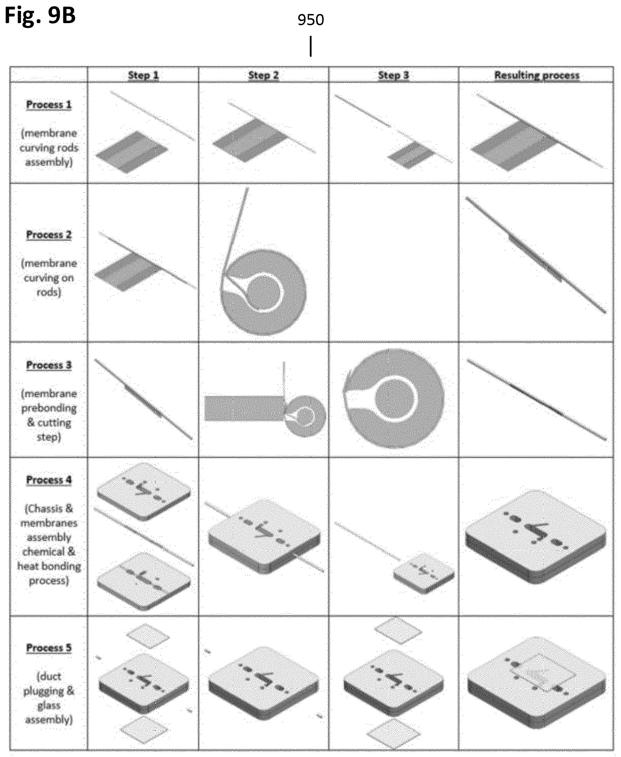

Fig. 9B illustrates the steps and processes, according to method 950, to execute the bonding process of the biochip 100 components where the ductal scaffold is round with no membrane extensions, which is pre-bonded in an area on the ductal scaffold surface along its length before assembling it to the chassis. In an example, the method may include, the curving process of the hydrophobic and hydrophilic membranes over a rod and bonding it on the ductal scaffold surface along its length using heat bonding. It then shows the assembly process of the membranes on the chassis. The method may also require taking the rod having the membranes bonded to it, locating it on the chassis, locating the hydrophobic flat stromal membrane in position, sandwiching everything between the two chassis, then binding them using chemical aided heat-press bonding. In this design, the stromal microfluidic channel void surrounds the ductal scaffolds on the chassis from all sides. Finally, this method may include the rod removal process, plugging the extremities of the ductal channel beyond the inlet and outlet holes, and assembling the coverslip glasses on both surfaces of the biochip 100.

### Method 5: Post-Bonding Membranes with Extension and Axial Tension:

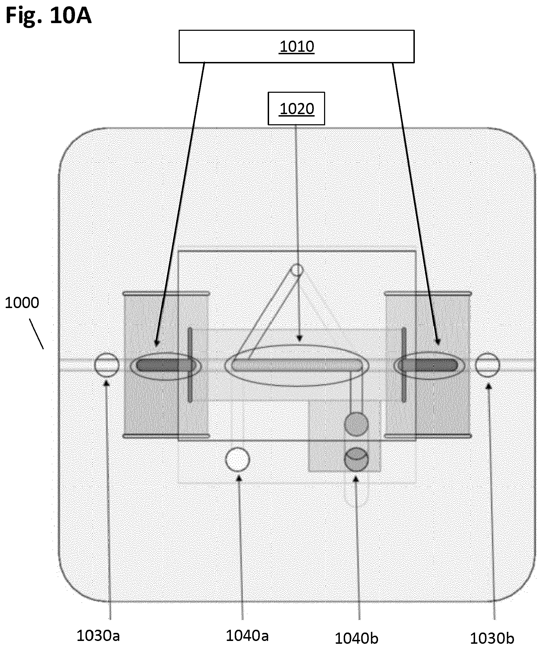

Fig. 10A illustrates a top view of the biochip 100 configured according to a method where two membranes are pulled after being curved each into a hemi-duct when assembled to apply axial tension. In an example, the biochip 1000 configured according to this method may include, air filtering area 1010, a duct surrounded by stroma 1020, a duct inlet 1030a, a duct outlet 1030b, a stroma channel inlet 1040a and a stroma channel outlet 1040b.

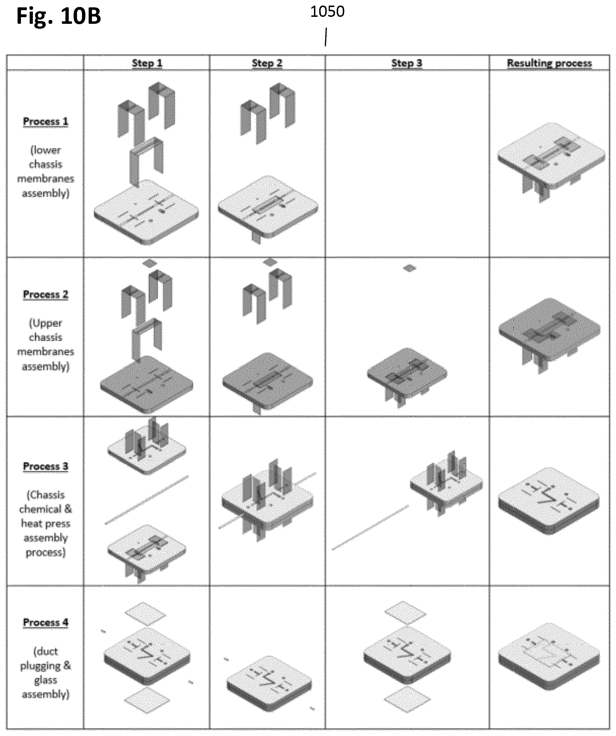

Fig. 10B illustrates the steps and processes, according to method 1050, to execute the bonding process of the biochip components where the ductal scaffold is made of two membranes aligned along with the chassis, pre-tensioned when sandwiched, and bonded post assembly to the chassis. In an example, the method may include the curving process of the hydrophobic and hydrophilic membranes over a rod placed on one of the chassis and pulling its extremities from holes aligned along the ductal scaffold. Additionally, the method may include locating the hydrophobic flat stromal membrane in position, compiling all members between the two chassis, then binding the membranes using chemical aided heat-press bonding. Finally, the method may include the rod removal process, cutting the membrane extremities, plugging the extremities of the ductal channel beyond the inlet and outlet holes, and assembling the coverslip glasses on both surfaces of the biochip 100.

### Method 6: One Chassis Chip with Fully Round Membranes without Extensions

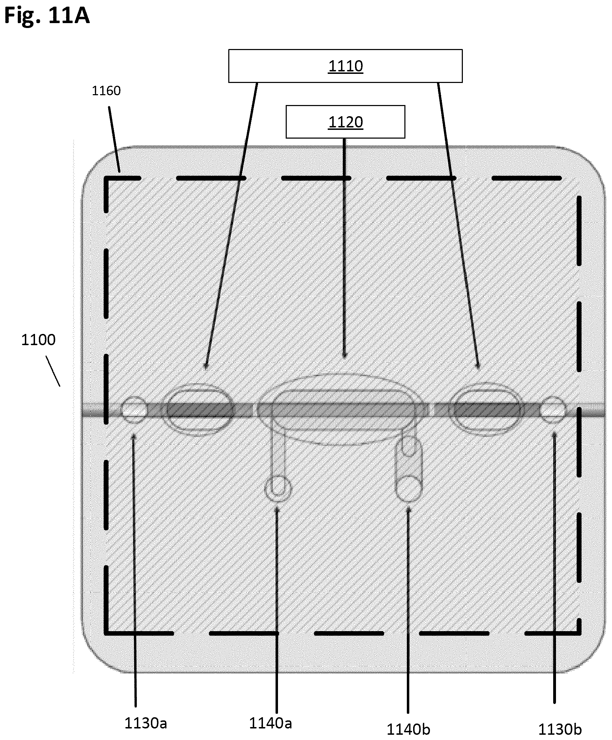

Fig. 11A illustrates a top view of a biochip 1100 configured according to a method where the two membranes are along its length with the membrane bonded and its extensions cut when assembled in only one chassis according to an aspect of the present disclosure. In an example, the biochip 100 configured according to this method may include, air filtering area 1110, a duct surrounded by stroma 1120, a duct inlet 1130a, a duct outlet 1130b, a stroma channel inlet 1140a and a stroma channel outlet 1140b. In an example, biochip 1100 includes a selectively removable material 1160 that is removed in response to one of UV radiation exposure, chemical etching or other stimulus.

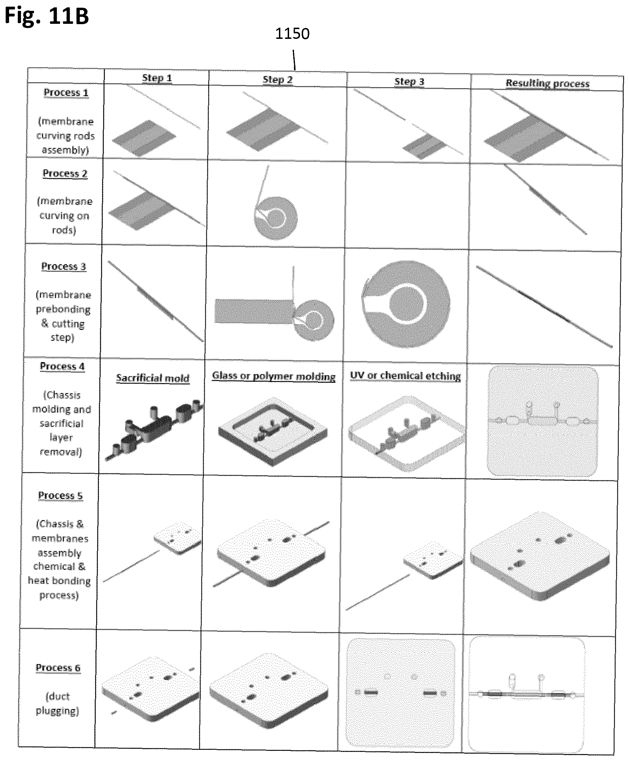

Fig. 11B illustrates the steps and processes, according to method 1150, to execute the bonding process of the biochip 100 components where the membrane is curved into a 180 degrees rotation, bonded in an area on the ductal scaffold surface along its length and the ductal scaffold extensions are cut prior to assembly in only one chassis. In an example, the one chassis contains the features of both chassis. In an example, the cylindrical void in the single chassis embodiment has a diameter larger than the cylindrical void in a two chassis embodiment. The chassis is formed by molding over a selectively removable material that is then removed in response to one of UV radiation exposure, chemical etching or other stimulus. The curved cylindrical membranes are placed on a rod and are inserted into position into the chassis void. Upon insertion in the chassis void, the whole chip (chassis-membrane assembly with the rod inside) is chemically bonded and heat pressed to deform the chassis and bond the internal features together. In an example, the last step of the disclosed method is to plug the duct extremities. In an example, coverslips are not utilized and in the chassis includes features capable of covering the plurality of apertures in the single chassis. In an example, the single chassis method utilizes a chassis made of a glass material.

### Method 7: One Chassis Chip with Fully Round Nested Membranes

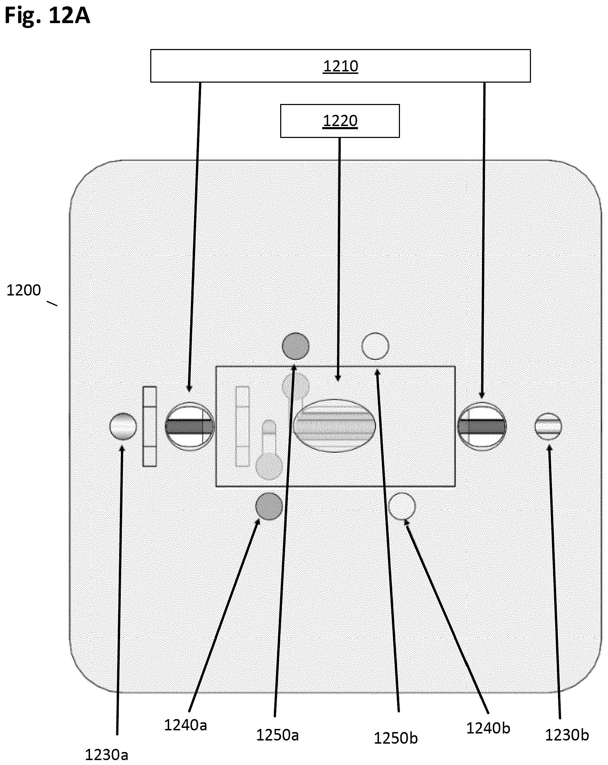

Fig. 12A illustrates a top view of the biochip 100 configured according to a method where the ductal scaffold is round with no membrane extensions and nested within another ductal scaffold, where each is pre-bonded in an area on the ductal scaffold surface along its length before assembling it to the chassis and could be accessed separately. In an example, the biochip 100 configured according to this method may include, air filtering area 1210, a duct surrounded by a duct and then by a stroma 1220, an inner duct inlet 1230a, an inner duct outlet 1230b, an intermediate duct inlet 1250a, an intermediate duct outlet 1250b, a stroma channel inlet 1240a and a stroma channel outlet 1240b.

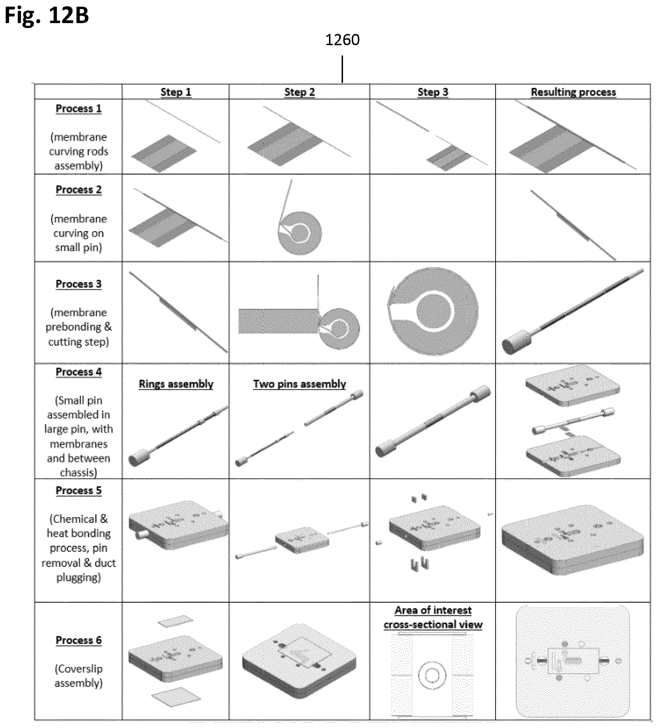

Fig. 12B illustrates the steps and processes, according to method 1260, to execute the bonding process of the biochip 100 components where the ductal scaffold is round with no membrane extensions and nested within another ductal scaffold, where each is pre-bonded in an area on the ductal scaffold surface along its length before assembling it to the chassis. In an example, the method may include, the curving process of the hydrophobic and hydrophilic membranes over a small pin equal to the diameter of the inner duct and bonding it on the ductal scaffold surface along its length using heat bonding. Additionally, the method may include the assembly of two plastic rings on the edges of one of the hydrophobic membranes and at one end of the hydrophilic membrane. In an example, the method also includes the assembly of another larger hydrophilic membrane on a larger pin of an inner diameter larger than the outer diameters of the rings and an outer diameter equal to the diameter of the outer ductal scaffold. The method may also include the assembly of the small pin with its membranes inside the larger pin. It then shows the assembly process of the pins with the membranes on the chassis, including the flat membranes along the channels leading to the stroma channel and the intermediate duct. The method may also require taking the rod having the membranes bonded to it, locating it on the chassis, locating the hydrophobic flat stromal membrane in position, sandwiching everything between the two chassis, then binding them using chemical aided heat-press bonding. In this design, the stromal microfluidic channel void surrounds both the inner and intermediate ductal scaffolds on the chassis from all sides. Finally, this method may include the rod removal process, plugging the extremities of the inner ductal channel beyond the inlet and outlet holes, and plugging the intermediate duct extremities by two puzzle-like parts from above and below getting in contact and surrounding the plastic rings, and assembling the coverslip glasses on both surfaces of the biochip 100.

In another embodiment, a method for assembling the biochip 100 is disclosed. According to the disclosed method, first clean and run quality checks on the manufactured chassis. This is accomplished by separating the chassis into the most accurate combination (top and bottom chassis). Use ultra-precise calibration rods to do so. Next, cut the 7um thick porous membranes into 40x3mm sheets (width error +/-0.1mm). Thoroughly clean the chassis by submerging them into a 2% sodium dodecyl sulfate (SDS) solution in distilled water; sonicate for 5 minutes dip the chassis into distilled water; sonicate for 5 minutes. Finally, clean the 2x 4cm membranes using the same method as the step above.

Next, the membranes are cut, positioned and fixed. Place the membranes perfectly symmetric above the hemi-duct chassis. Stretch the membrane (tensile force, duct axial direction) and fix the extremities on each of the chassis on the top surface of the chassis using a drop of 1uL chloroform. Place the top chassis with membrane assembled against a sacrificial surface with the membrane between the chassis and the surface, fix it and drill the membrane through the chassis inlet/outlet holes. Next, locate the lower chassis on the chip holder designed to be placed inside the heat press device. Locate the 0.5mm diameter wire or the ultra-precise 0.5mm diameter steel rod in position above the hemi-duct of the bottom chassis. Place 1mm diameter 2.5mm long steel pin in the lower chassis stroma channel inlet hole (this will help assemble the two subassemblies precisely above each other regardless to the relatively large laser cutting errors at the edges, since the CNC machine precision (same tool and chip holding) is less than 5um. Place 0.5mm diameter 2.5mm long nylon pin in the lower chassis connecting hole (this in addition to the wire positioning and the other pin, will help assemble the two subassemblies precisely above each other regardless to the relatively large laser cutting errors at the edges, since the CNC machine precision (same tool and chip holding) is less than 5um.

Continuing the disclosed method for assembling the biochip 10, pipette 10uL of 20% chloroform in ethanol homogeneously on each of the chassis interfacing surfaces. Directly press the items together and close the chip holder. Instantly place under the heat press and apply a 53Mpa stress at 82C for 7 min. Quench the chip in distilled water and keep for 30 sec (to prevent membrane crystallization). Next, once the clamping process is complete, connect the variable collet to the visible and accessible region of the 0.5mm steel rod or the wire which is stuck inside the chip. It should be noted, this rod is used to accurately deform the membrane into the correct features. Remove the rod/wire from the duct by holding the collet and pulling to the opposite direction of the clamp or by using a rod to push the collet against the clamp. Next, the duct extremities must be plugged. Cut 1.5mm long nylon wires having diameter = 0.5mm and use them as plugs, by placing them inside the duct channel at the extremities of the duct beyond the inlet/outlet holes. Drop 1 mm pure chloroform on the plug after inserting it in the duct extremities which is enough to melt the PMMA surrounding the nylon plug and seal them together.

In addition to the aforementioned steps of the disclosed method, run leakage testing from the duct by pipetting 10uL of colored water into the duct. If leakage occurs under the membrane, then the duct grooves are cut shallow into the chassis. If the leakage occurs directly from the duct channel to the stroma channel, then the leakage occurs because the duct grooves are cut deep into the chassis. If both leakages happen, then the leakage is because the stromal channel is cut deep under the flat surface, and low pressure of binding occurs there. Conduct pore diffusion testing, by inserting colored water into the duct, and by using a tissue wipe for delicate surfaces, for example a Kimwipe^{®}. Insert the tissue wipe from the stromal region and let it touch the porous duct's outer surface. If the diffusion occurs from the duct to the tissue wipe and the tissue wipe absorbs from the colored water, then the pores aren't blocked.

Next, the cover coverslip assembly must be assembled. First, use 2 coverslips (1×1cm) and place each on either sides of the assembled chip. Next, pipette 2uL of acrylic acid glue dispensed homogeneously on the bounding region, to bind the coverslips to the PMMA surface. Finally, wait for 3 hours for the glue to cure. Dual flow testing must be conduct. In order to do so, flow colored water through both channels of the chip (in each channel pass a different color). A mixture between the two colors should happen due to diffusion through the pores. Finally, no leakage should occur for both channels.

The final steps of the disclosed assembly method are to clean the LOC by dropping it into a 2% SDS solution in distilled water, then sonicate for 5 min. Then dip it into distilled water, and sonicate for 5 minutes. Package each chip in a blister and close the blister by a sterilization Tyvek sheet, and seal it using the heat press machine. Take the chip to the H2O2 sterilization facility to sterilize it. Finally, package each 12 blisters in a box having the logo.

In another embodiment, a method for assembling the biochip 10 that utilizes automation is disclosed. First, the machine being utilized holds the lower chassis on a moving plate at a fixed relative position through 2 projecting 2.5mm long pins (1mm and 0.5mm in diameter) that goes into the lower chassis two holes. Next, directly above the lower chassis holding plate, the machine holds the chassis on a moving plate at a fixed relative position through 3 projecting 1mm long pins (1mm diameter) that goes into the upper chassis two inlet and outlet holes (except the stroma channel inlet hole). Next, the moving plate goes to a chamber where a feed of 2 (7um thick) porous membranes 3mm wide are being cut into shape but extended length. Both are pushed against the chassis hemi-ducts. One is pushed upward against the upper chassis, and the other pushed downwards against the lower chassis hemi-duct. Next, after locating the membrane in position, a drop of 1uL of pure chloroform will be dropped to the membrane extremities while it is pushed and tensioned and then the membrane is cut.

Continuing the disclosed automated assembling method, the two subassemblies (upper and lower chassis with membranes) seed into a position where its surfaces are to be sprayed with 10uL of 20% chloroform diluted in ethanol. Next, the two sprayed subassemblies are fed into a position were an ultra-precise steel rod is sandwiched in between and then clamped under a heat press for 7 minutes. After the 7 minutes, the rod will be automatically removed, and the top plate that was at first connected to the top chassis was also removed giving a motion degree of freedom to the chip. While the chip is still in position, and two nylon plugs are fed to close the duct extremities beyond the inlet/outlet holes, and 1uL of chloroform is dropped into them. A quality control step is done here, where colored water is to be passed through the duct of the chips, and a computer vision program will check for failures. The failed chips will go to a failure collection container while the ones that succeed will go to a sonicating and cleaning step. After the cleaning step, 2uL of acrylic acid glue is dispensed homogeneously across the glass coverslips and then the two coverslips are pushed against the chips' surface for 3hrs to bind it. The first coverslip to get in position is the top one where a plate containing three pins will also hold the chip upward against the top glass coverslip while the bottom pins connectors are removed. Then, the chip will be moved to another plate containing 2 pins and the coverslip glass with glue.

Finally, another quality test is done here by-passing two-colored fluids into the two channels of the chip, and separating through a computer vision program. If failure occurs, also the chips will go to another failure collection container. The ones that succeed will proceed to a final cleaning step in a sonicating bath. Next, the chips are then dried by 50C airflow and are seeded automatically to the blisters. The blisters are then covered with the Tyvek sheet and bonded together using a heat press step. The blisters containing the chips are now ready to send to a sterilization facility. Finally, after getting back from the H2O2 autoclave, the blisters are packaged with the rack in a box of 12.

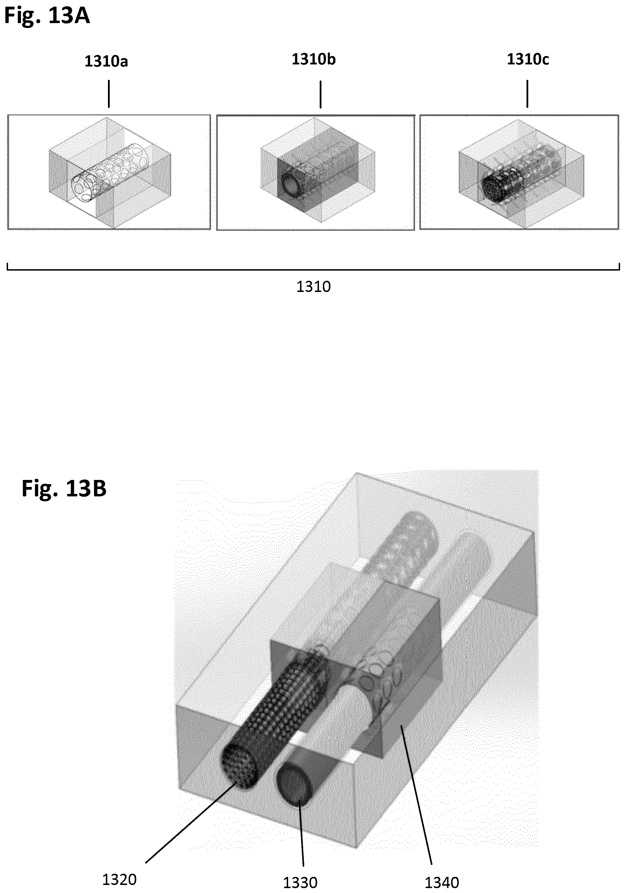

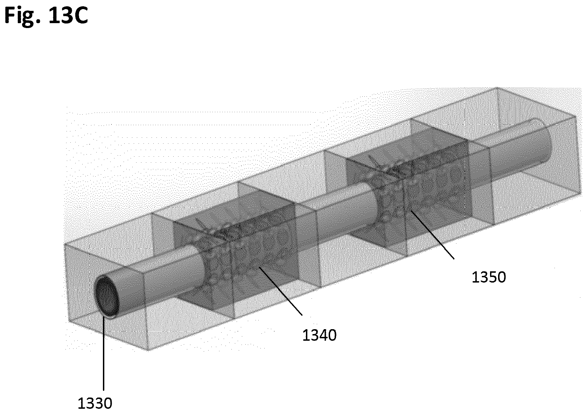

Fig. 13A, 13B and 13C illustrate possible embodiments of the biochip according to aspects of the present disclosure. Fig. 13A illustrates additional possible uses of the biochip according to the aspects of the present disclosure. In an example, 1310a shows the biochip empty because no cells have been cultured within the biochip. Additionally, 1310b shows the biochip cultured with vascular tissue surrounded by stromal tissue grown inside the biochip. Furthermore, 1310c illustrated a biochip cultured with ductal epithelial tissue surrounded by stromal tissue grown inside the biochip, according to an aspect of the present disclosure. Fig. 13B illustrates an example embodiment of the biochip, where one external compartment 1340 surrounds two ductal scaffolds 1320 and 1330. For example, stroma tissue is grown in the external compartment 1340 surrounding both ducts 1320 and 1330. Inside one of the ductal scaffolds 1330, the vascular tissue is grown and attaches to the porous duct walls. Inside the other ductal scaffold 1320, ductal epithelial tissue is grown and attaches to the porous duct walls, according to an aspect of the present disclosure. Fig. 13C illustrates an example embodiment of the biochip, where two separate external compartments 1340 and 1350 surround one ductal scaffold 1330. For example, two different stromal tissues grown in each of the two external compartments 1340 and 1350 surround a duct growing a blood vessel.

### SEEDING THE BIOCHIP

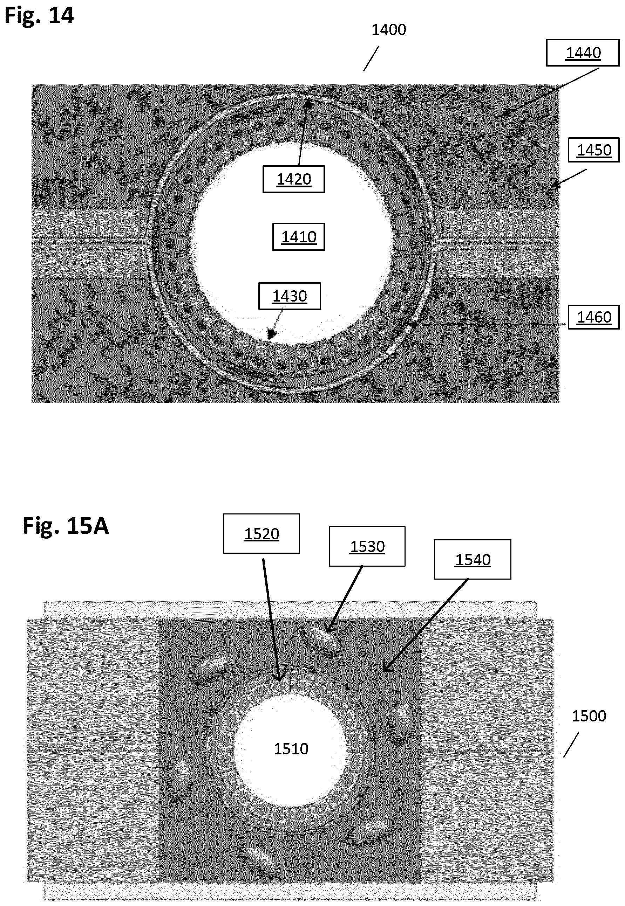

Fig. 14 illustrates a circular duct morphology tissue microstructure of epithelial cells grown on the porous membrane of the cylindrical duct of the biochip 1400, according to an aspect of the present disclosure. The porous membrane of the cylindrical duct of the biochip 1400 includes a duct channel 1410, a porous member 1420, epithelial cells 1430, stroma channel 1440, stroma tissue 1450 and Myoepithelial cells 1460. In an example, the epithelial cells may be one of renal, hepatic, lung or breast ductal epithelial cells. In an example, the Stroma tissue 1450 may be one of fat cells, fibroblasts which may be incorporated in a hydrogel. In an example, the Myoepithelial cells 1460 may be incorporated in a basement membrane hydrogel. Additional examples of epithelial cell and stroma tissue that may be utilized are discussed in a later section of this disclosure.

In another embodiment, a method for seeding cells in the duct channel 1410 against extracellular matrix (ECM) gel for full duct formation in the biochip 1400 is disclosed. Tubular structures, such as endothelial or epithelial barrier tissues, are established in the chip 1400 by growing cells in the duct channel 1410 against an ECM gel in the stroma channel. Morphology and function of the tubule can be assessed by microscopy, a barrier integrity assay, or other functional assays. In addition, cells, media and ECM from each channel 1310 could be removed separately for component assessment such as qPCR, a property unique to chip. In an embodiment the following materials are necessary to perform the disclosed seeding method: chip 1200; rack (12-well plate format - square wells); collagen-I 5 mg/mL (AMSbio Cultrex^{®} 3D collagen I rat tail, 5 mg/mL, #3447-020-01); 1 M HEPES (Life Technologies 15630-122, pH 7.2-7.5); 37 g/L NaHCO3 (Sigma S5761-500G, dissolve in sterile water, adjust pH to 9.5 using NaOH); Medium (12.5 mL per rack (12 chips)); cells: seeding density is dependent on the cell type; 10uL or 20uL pipettes (ex: Eppendorf Research^{®} plus (single-channel, variable volume)); 1mL pipette (any type) (optional); 10 or 20uL pipette tips (e.g. epT.I.P.S.^{®} Standard, Eppendorf Quality^{™} 0.1-10uL or 0.1- 20uL); Medium reservoir; 1mL pipette tips (any type) (optional); sterile tweezer (small size); and crushed ice.

Referring to FIG 13, according to the disclosed method, the seeding method begins with opening the sterile blisters containing the rack and chips 1300 under the biosafety cabinet and placing the chips in the rack (up to 12 chips per rack). Next, add 14 µL of HBSS to each chip's stroma channel using a 10uL or 20uL pipette. Next, prepare the required amount of ECM gel (e.g.14 µL gel per chip) according to the following Collagen-I 4 mg/mL preparation method: place an Eppendorf tube on ice; the collagen-I 4 mg/mL gel is prepared by mixing 1 M HEPES, 37 g/L NaHCO3, and 5 mg/mL collagen-I in a 1:1:8 ratio; prepare at least 100 µL of total gel volume to ensure components mixing; mix well by pipetting the mixture up and down >20 times, while on ice ; if bubbles are formed, quickly spin the tube down (~5 seconds); and Use gel immediately after preparation (within 10 minutes). Next, using the 10 or 20uL pipette, fit the tip of the pipette inside the duct inlet hole 26. Make sure the tip fits well (should stick in the hole), then gently dispense 14uL. Use the tweezer to hold the chip in place before removing the pipette. Dispense 14uL gel into each chip's stroma channel inlet using the 20uL pipette.

Next, place the rack containing the chips in a humidified incubator (i.e. 37°C, 5% CO2) for 15 minutes to allow polymerization of the collagen-I gel. Add a 2 µL drop of HBSS above the ECM channels inlet & outlet to prevent the gel from drying out. Here you are free to seed the epithelial cells at any time, not necessary directly. Harvest cells according to their dissociation protocol. Count the number of live cells in the cell suspension. Calculate the required number of cells for seeding in the chips and pellet them. In an embodiment, the optimal cell density is cell type dependent (generally between 1,000 and 10,000 cells/µL). Re-suspend pellet in [900,000/ 10,000 =] 90 µL medium to obtain a 10,000 cells/µL cell suspension. Seed 6 µL of cell suspension in the duct inlet using the same pipetting procedure as previously used for gel loading. Re-suspend the cell suspension during seeding to ensure homogenous cell density. To ensure the cells attach on both sides of the duct (full circular duct formation), a seeding or post seeding process is also needed (described in next section).

In an embodiment, the biochip 1400 can be flipped to allow cellular growth to form a full duct with the duct interface 1410. The biochip 1400 can be placed on a rack in an incubator on its top surface (top surface down) until a layer of cells attach on the top surface. The biochip 1400 is then gently flipped. Next, 1mL of media is added to the biochip 1400. The biochip 1400 is then covered and placed back in the incubator. The time cells need to attach is cell type dependent and generally varies between 30 minutes to 6 hours. Cells contained in the duct 1410 are sufficient to cover both surfaces. Therefore, placing the rack upside down is adequate for the cells to settle and attach on the top surface. After flipping the biochip 1400, the excess cells not attached to the top surface will settle to the bottom surface and attach. Finally, 1mL of media is added in each rack well containing a chip 1400 to prevent the drying of the media and the gel inside the channels.

In another embodiment, an additional method can be utilized before seeding the cells by coating the inner surface of the duct by dispensing diluted collagen IV (5ug/mL of media) into the duct to aid cell attachment, then flow the suspended cells in media at a very low flow rate into the duct and move delicately to the incubator. Next, pipette 6uL of the 5ug/mL collagen mixture in media and place dishes with chips in 37 °C incubator. After coating with type IV collagen, the chips are ready to be seeded with cells or they may be stored for up to one week at 4 °C. Use a 5uL Hamilton syringe needle to gently dispense the cells into the duct. Using extreme caution, move it delicately to the incubator. According to this procedure, cells flowing into the duct will attach homogeneously to the walls of the duct and surface tension of the collagen will be enough to hold the cells in position if no shaking occurs.

In another embodiment, seeding the cells into the duct can be achieved by using a micro-syringe pump at decreasing flow rates, to ensure homogeneous attachment. First, clean the butterfly needle tigon tubing by flushing 70% ethanol into it, keeping it to dry and then wash it with media. Next, connect a multi-headed micro-syringe pump to the inlets of the chips using the cleaned tigon tubing and a 10uL pipette tip fitted to the chip's inlet. Similar to other disclosed procedure of a flow is to be executed into the duct 1st at flow rate of 10uL/min (found from CFD simulation) for 5 min, to ensure the proper flow pressure needed to push the cells against the duct walls. Reduce the flow rate to 1uL/min after 5 min and keep it for 50 min. Finally, remove the pump connections and place in the incubator.

In another embodiment, after pipetting cells, connect the rack to a rotary rocker designed to ensure homogeneous ductal monolayer. The rotary rocker is designed to rotate the chip around the duct axis at a controlled speed.

In another embodiment, a method for changing the medium of cultures grown in the biochip 1400 is disclosed. Most cultures in the chip 1400 require medium refreshment every 2-3 days. Old medium can be pipetted outside of the channels or aspirated using an aspirator system. After the channels are emptied, fresh medium can be added using a pipette. The disclosed method allows for medium changes in the chip 1400 and can also be used for other assays, such as fixation. In an embodiment, the materials necessary for utilizing the disclosed method are as follows: chip 1400; rack (12-well plate format - square wells); cell specific medium; aspirator system and tips (optional); 10uL or 20uL pipettes (ex: Eppendorf Research^{®} plus (single-channel, variable volume)); 1mL pipette (any type) (optional); 10 or 20uL pipette tips (e.g. epT.I.P.S.^{®} Standard, Eppendorf Quality^{™} 0.1-10uL or 0.1-20uL); medium reservoir; 1mL pipette tips (any type) (optional); and sterile tweezer (small size).

In an embodiment of the present disclosure, medium changing can be accomplished by retrieving a rack containing the chips from the incubator, placing the rack under the biosafety cabinet and remove the rack lid. Next, aspirate the medium from the well containing the chip using a 1mL pipette or using aspirator. Next, aspirate the medium from the inside of the channels a. Use the 10 or 20uL pipette, fit the tip of the pipette inside the duct inlet hole. Make sure the tip fits well (should stick in the hole). Aspirate 10uL of the medium which is excess volume. Use the tweezer to hold the chip in place before removing the pipette. Next, dispense 6uL of the new medium into the inside of the channels (same as step 3). Repeat the previously disclosed steps all the chips 1400 used in the rack (up to 12 chips). Next, dispense 1mL of medium above each chip 1400. Finally, close the rack lid and place it again in the incubator.

### EXAMPLES OF TISSUE CULTURED

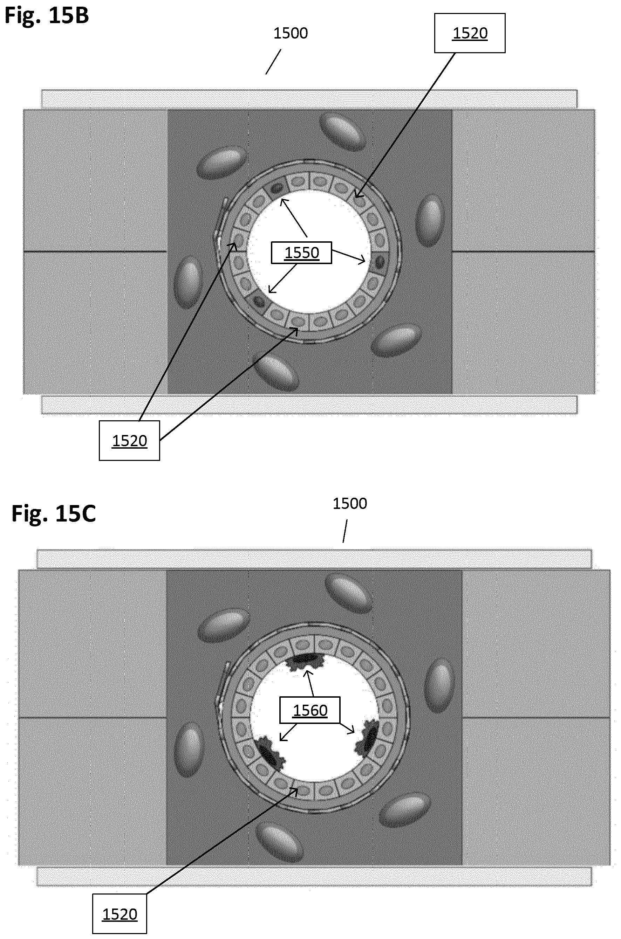

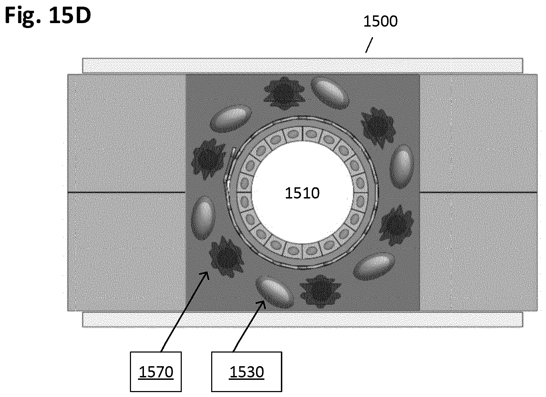

Fig. 15A, 15B, 15C and 15D illustrate different embodiments utilizing biochip 100 to culture various cell types.

Fig. 15A illustrates a cross-sectional view of the ductal tissue model utilizing a biochip 1500 according to the present disclosure. The ductal tissue model includes a duct channel 1510, endothelial cells 1520, primary human hepatocytes 1530 and stromal gel 1540. The endothelial cells 1520 may be one of human liver-derived endothelial cells (HLEC). In some examples, the primary human hepatocytes 1530 may be primary human glomerular microvascular endothelial cells.

In an another example, the ductal tissue may be one of hepatic, renal, pulmonary, mammary, pancreatic, prostatic, vascular, lymphatic, glandular, or other types of tissue.

In some examples, the stromal gel 1540 may be stromal gel associated with the specific endothelial cells cultured may be used. For example, if renal proximal tubule cells (RPTEC) are utilized, renal stromal gel can be used. The ductal tissue model is able to be used where disease-free normal epithelial or endothelial cells are grown attached to the inner walls of the internal compartment of the porous ductal scaffold. Also it shows a stromal tissue grown in the external compartment of the ductal scaffold, according to an aspect of the present disclosure.

In an embodiment, stroma can be customized by the user to be made of biopsies, gels or biological materials with various components and viscosities. In an example, stem cells could be grown in the ducts forming a hollow duct or a sausage-like structure where cells could be nourished from the stromal channel. In another example, the cells and biological materials could be extracted from biopsies, cell lines and other biologically relevant materials.

Fig. 15B illustrates the biochip 1500 used to culture cancerous cells. The biochip 1500 may be used to culture cancerous cells 1550 near disease-free normal epithelial or endothelial cells 1520 to study a tumor tissue disease model. In an example, the cancerous cells 1550 or previously grown solid-tumor clusters are inserted at the same time in the internal ductal scaffold compartment.

Fig. 15C illustrates the biochip 1500 used to culture solid-tumor clusters. The biochip 1500 may be used to culture solid-tumor clusters 1560 near disease-free normal epithelial or endothelial cells 1520 to study a tumor tissue disease model. In an example, the solid-tumor clusters 1560 are inserted at the same time in the internal ductal scaffold compartment. This allows for creating a disease model for metastasized tumors.

Fig. 15D illustrates an embodiment of biochip 1500 utilizing a tumor tissue disease model where disease-free normal stromal tissue 1530 is mixed with cancerous cells 1570 or previously grown solid-tumor clusters, and inserted at the same time in the external ductal scaffold compartment, surrounding the duct 1510, and creating stromal or parenchymal based disease model, according to an aspect of the present disclosure.

In an embodiment, the biochip 1500 may be used as a hepatotoxicity drug testing model, where primary human hepatocytes are cultured within the hepatic stroma gel and grown in the external compartment of the duct. Inside the duct, Human liver-derived endothelial cells, which include hepatic microvascular cells, are grown and attached to the ductal scaffold inner wall, according to an aspect of the present disclosure.

In an embodiment, the biochip 1500 may be used as a nephrotoxicity drug testing model, where primary human glomerular cells and primary human glomerular microvascular endothelial cells 1520 are cultured within the renal stromal gel 1540 and grown in the external compartment of the duct. Inside the duct 1510, Human renal proximal tubule cells are grown and attached to the ductal scaffold inner wall.

In an embodiment, the biochip 1500 may be used as a toxicity drug testing model where the liver's hepatotoxicity model (which also metabolizes the drug) is micro-fluidically connected to the renal nephrotoxicity model, according to an aspect of the present disclosure.

In an embodiment, the biochip 1500 may be used in a model for testing lung cancer drug efficacy. The human lung fibroblasts are cultured within the pulmonary fibroblastic stromal gel 1440 in the external compartment of the duct. Inside the duct, human small airway epithelial cells for the air-liquid interface are grown attached to the inner walls of the ductal scaffold. On the luminal surface of the epithelial cells, solid tumors of the KRAS positive human lung adenocarcinoma cells or non-small cell adenocarcinoma cells are attached and grown, according to an aspect of the present disclosure.

In an embodiment, the biochip 1500 may be used as a model for testing prostate cancer drug efficacy. The human prostate stromal cells are cultured within the prostate stromal gel in the external compartment of the duct. Inside the duct, the human prostate epithelial cells are grown attached to the inner walls of the ductal scaffold. On the luminal surface of the epithelial cells, solid tumors of the LNCaP clone FGC prostate carcinoma cells or NCI-H660 stage E prostate cancer small cell carcinoma cells are attached and grown, according to an aspect of the present disclosure.

In an embodiment, the biochip 1500 may be used as a model for testing Breast cancer drug efficacy. The human primary mammary fibroblasts are cultured within the mammary stromal gel in the external compartment of the duct. Inside the duct, the human mammary epithelial cells are grown attached to the inner walls of the ductal scaffold. On the luminal surface of the epithelial cells, solid tumors of the HCC1500 (Estrogen +ve & Progesterone +ve) or HCC70 (Estrogen +ve) or HCC2157 (Estrogen -ve & progesterone +ve) cells are attached and grown, according to an aspect of the present disclosure.

In an embodiment, the biochip 1500 may be used as a model for testing brain cancer drug efficacy across the blood-brain barrier. The human astrocytes are cultured within the brain astrocytic stromal gel 1540 in the external compartment of the duct. Inside the duct, the human brain cerebral microvascular endothelial cells are grown attached to the inner walls of the ductal scaffold. On the luminal surface of the vascular cells, solid tumors of the U-118 MG brain glioblastoma & astroblastoma grade IV tumor cells are attached and grown, according to an aspect of the present disclosure.

In an embodiment, the biochip 1500 may be used as a drug testing model for drug dosage according to the toxicity and efficacy balance. The drug dosage model consists of the microfluidic connection of the hepatic model, renal model, and then the disease model. In this model, the drug passes first through the hepatic chip, where the drug gets metabolized and tested for hepatotoxicity. Then the drug passes through the renal chip to test for toxicity in the renal proximal tubule. Then, the drug passes through the disease model to test for drug efficacy on the grown tumors. This illustration shows the dosage model for the lung, prostate, breast, and brain cancer drugs. The drug usually succeeds the trial if any optimal drug balance for efficacy and toxicity is discovered, according to an aspect of the present disclosure.

In an embodiment, the biochip 1500 could be used by researchers to study the physiology and pathology of ductal tissues, the mechanotransduction effects on ductal tissues, signaling pathways in ductal tissues, preferential cancer metastasis through ductal tissues, the diffusion and effect of drugs, toxicant and biological stimulants on ductal tissues and for the mass generation of stem cells.

In an embodiment, the biochip 1500 could be used by pharmaceutical companies to grow ductal organoids, and human-on-chip models to test drugs on it and study its safety, efficacy and dosage to better predict the drug reaction in clinical trials

In an embodiment, the biochip 1500 could be used by clinical researchers for precision and personalized medicine, by growing ductal organoids from patient biopsies, creating a personalized patient-on-chip, to test different treatments and drugs on it and prognose a precise and personalized treatment.

In an embodiment, the biochip 1500 may include ductal scaffold that restricts the ductal epithelial and endothelial tissues assembly and growth into just a monolayer attached to the inside walls of the ductal scaffold, taking its shape. The cylindrical structure of the ductal epithelial tissues is linked to preserving the cell polarity through the differentiation markers and junction proteins on the membranes of the cells. Growing the epithelial or endothelial ductal tissues in cylindrical structures inside the biochip, allows the tissue model mimetic to the in vivo tissues.

In an embodiment of the biochip 1500, the external compartment and the pores of the ductal scaffold allow the culture and growth of any 3D tissue in suspension or in a gel to be interfaced with the ductal tissue inside the duct. Co-culturing epithelial cells with the surrounding stroma is important because fibrous tissue plays a vital role in the ductal cancer progression through hormonal and growth factor level fluctuations. Such fluctuations cause changes in the stromal cells' gene expression, leading to different extracellular matrix biomarkers, and thus disrupting the signaling cascades from and to the epithelial tissues. Co-culturing the epithelial or endothelial ductal tissues with its corresponding stromal tissues, makes the tissue model mimetic to the in vivo tissues.

In an embodiment of the biochip 1500, the porous-walled cylindrical ductal scaffold, having both an external & external compartment, allows the precise access of the cells, tissues and extracellular matrices from any specific location without disrupting the other tissues. This allows the controlled placement of tumor cells or solid tumor clusters in any location across the model for a specific tumor disease model. The resulting effects of the drugs tested on tissues grown inside the biochip depends on the precision in modeling the tumors size, density and location. Having a ductal scaffold makes the tumor disease model mimetic to the in vivo tissues.

In an embodiment of the biochip 1500, the pore size, shape and density in addition to the thickness of the wall of the cylindrical ductal scaffold allows cells squeeze and pass through the wall if subjected to any signaling pathway. This mimics the cell migration process across tissues.

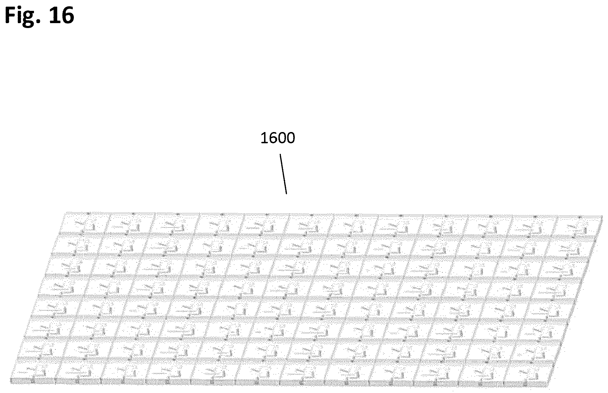

Referring to FIG. 16, in another embodiment, the disclosed biochip 100 may include multiple ducts and multiple surrounding channels. In at least one aspect of the present disclosure, the biochip may be configured in a 96-well plate design with multiple parallel ducts so that it can be used for high throughput drug screening purposes. In such an aspect, the system has the dimensions of a standard 96-well plate and, in some instances, can be used with a pump connected to the inlet and outlet port. In other instances, fluid can be pipetted manually into the inlet port and it will fill the entire duct with the help of capillary forces.

In an example, the biochip 100 may have multiple design configurations where multiple ducts could be designed to be interfaced with one or more surrounding channels to be used for drug diffusion testing applications.

In an example, the biochip 100 may have multiple channels that could be designed to be interfaced with one or more ducts, to be used for cell migration and metastasis applications.

In an example, the biochip 100 may be configured to interact with multiple chips containing different types of tissue and organoid systems, sourced from the same or different individual or cell-line type. For example, a plurality of biochips 100 can be connected to form a human-on-chip mode.

### METHOD FOR MANUFACTURING BIOCHIP

In another embodiment, a method for manufacturing the biochip 100 is disclosed. According to the disclosed method, Poly(methyl methacrylate) (PMMA) is cut into desired shape and thickness using a laser cutting machine. First, a sheet of 1.5mm thick PMMA is gathered and the thickness of the sheet at different regions is inspected using a thickness caliper. For a sheet 300x300mm, it should not vary between the two sides more than +/-0.1mm. Next, a sacrificial step on surplus material is dine to optimize on the laser cutting process. The thickness of the laser beam must be taken into account. For example, for a specific machine to cut a 2cm x2cm square, the input dimensions may be 2.1x2.15cm (taking the machine axis error difference). The protective layer coating of the PMMA sheet should remain on at all times during the manufacturing process. Next, the whole PMMA sheet is cut into equal 2x2cm squares (note: end result should be 2×2cm). According to this method, 30x30cm sheet should give at least 196 PMMA squares. While still assembled in position in the laser cutting machine and using a marker, label the cut chassis either horizontal or vertical sides as a reference for next operations (to take into account the laser cutting errors for the next step). Further label each square using a marker on its edge (the same side labeled before, and remove the protective layer coating of the PMMA.

According to the disclosed method, the next step of manufacturing the biochip 100 is to assemble a custom-made upward piston clamp on an automated milling machine such as a computer numerical control (CNC) milling machine. First, place the upward clamp on the CNC machine and clamp it. Before fastening the CNC clamp handle to fix the upwards clamp, connect the touch probe to the CNC machine and optimize on the clamp top surface calibration according to the touch probe used. Next, iterate on the clamp position until ensuring that the top surface of the clamp is perfectly leveled, then fix the clamp and fasten it well. Next, place a square PMMA from the previous step on the clamp piston, and push up the piston until a grip is achieved and the 2cm × 2cm PMMA top surface is coplanar with the clamp top inner surface. This surface z-axis position will be the same regardless to the thickness error difference of the PMMA squares. Next, connect the 0.5mm diameter ball mill to the CNC machine and take the z-axis zero reference when the ball mill touches the assembled chassis surface. Further calibrate the clamp position, by trying to cut the first milling operation of the 0.5mm diameter, 0.25mm deep hemi-duct cut at the middle of the chassis throughout its length.

According to the disclosed method of manufacturing the biochip 100, the quality assurance step requires un-gripping the chassis piston and remove the chassis with this 1 operation cut. Repeat this for 20 different chassis. Next, conduct quality testing on the depth of cut, in an embodiment, using the ultra-precise precision rods from the two sets (high resolution 0.5mm diameter shaft (Plug Gauge Set No-Go and the Plug Gauge Set Go)). Next, place the 0.5mm rod in between two chassis hemi-ducts, and press this sandwiched assembly by hand. Try to remove the rod, if the rod was easily removed, then the hemi-ducts are cut deeper than 0.5mm, repeat the calibration. If the No-Go rod wasn't easily removed, then check if the two chassis surfaces align on each other well, if they didn't and there is a gap in between, then the hemi-duct depths are shallow, repeat the calibration. If the No-Go rod was not easily removed and there is no gap between the two manually sandwiched chassis, then there is no significant error in the depth of cut, thus, proceed to the Go-rod testing for further quality assurance. Next, place the 0.5mm rod in between two chassis hemi-ducts, and press this sandwiched assembly by hand. If the rod wasn't easily removed, then the duct is slightly shallow. Finally, if the Go-rod was easily removed, then the hemi-duct groove was cut within an error of +/-0.5um.

According to the disclosed method of manufacturing the biochip 100, the CNC operations are as follows with feed rates approximately 100mm/min and spindle speeds of 7000 revolutions per minute (RPM). Operation 1, using the same 0.5mm ball mill from the calibration process, cut a 0.25mm deep groove passing through the center of the chassis from side to side. Operation 2, automatically starts after operation 1, by cutting a 5mm long (along the hemi-duct groove cut in operation 1 at its center), 0.5mm wide groove throughout the chassis depth by 6 cutting steps (0.25mm deep each). Operation 3, automatically drill the 0.5mm connecting hole 4mm away from the duct axis center. Operation 4, for half of the chips and through the same tool and chip assembly as the 1st three operations, cut the 1.5mm long 0.5mm wide and 0.5mm deep channel (upper chassis). Operation 5, for half of the chips and through the same tool and chip assembly as the 1st four operations, drill the other 0.5mm connecting hole (upper chassis). Next, remove the chip from the clamp's piston, assemble another one and do the same for all the square PMMA sheets cut in the laser cutting operation.

According to the disclosed method of manufacturing the biochip 100, additional CNC operations are as follows with feed rates approximately 100mm/min and spindle speeds of 7000 revolutions per minute (RPM). First, flip chip to the other side, and using same tool proceed with following operations. Operation 6, cut the oblique 4mm horizontal length and 2.5mm vertical length 0.5mm wide and 0.4mm deep groove for all the chips. Operation 7, for half of the chassis (lower chassis), and using the same tool, cut the 4mm long, 0.5mm wide and 0.4mm deep horizontal groove from the stroma inlet hole to the duct interface edge. Operation 8, for the other half of the chassis (upper chassis), and using the same tool, cut the 2.5mm long, 0.5mm wide and 0.4mm deep horizontal groove from the stroma inlet hole to the connecting hole beside the stroma channel outlet. Operation 9: for the other half of the chassis (upper chassis), and using the same tool, cut the 2.5mm long, 0.5mm wide and 0.4mm deep horizontal groove from the stroma inlet hole to the connecting hole beside the stroma channel outlet. Next, remove the chip from the clamp's piston, assemble another repeat for all chips. Operations 9-12: assemble the 1mm drill, locate the upper chassis on piston and drill the four remaining inlet and outlet holes for the duct and the stroma channel. Finally, repeat all operations for all of the other upper chassis.

In another embodiment, an additional method for manufacturing the biochip 100 is disclosed. The biochip 100 can be manufactured utilizing injection micro-molding processes. To do so, first manufacture the cast using CNC machining. Several iterations and quality checks should be made to ensure the accuracy of the cut features in the molding cast. After optimizing on the cast manufacturing, the injection molding process is to be initiated, where the molding parameters are to be optimized to get accurate chassis features and dimensions where quality checks are done on it. Iterations should be made on the molding process to ensure we get repeatable chassis with acceptable resolutions.

## Claims

1. A method of manufacturing a biochip, the method comprising:
providing at least one chassis, wherein the at least one chassis includes,
a channel configured to support the at least one membrane structure and
at least one microfluidic channel in fluid communication with the channel;
providing at least one porous membrane;
curving at least one porous membrane into a closed loop of cylindrical cross-section.
wherein a round, cylindrical porous duct is formed by curving a first part of a porous membrane and a one or more additional porous membranes form a second part of the duct, and
inserting the at least one porous membrane between the at least one chassis.

2. The method of claim 1, wherein the at least one chassis is configured to contain features forming the microfluidic channels leading to the internal and the external compartments of the ductal scaffold.

3. The method of claim 1, wherein at least one membrane structure is combined within the internal space within the cylindrical scaffolds of the at least one membrane structure, providing layers of porous membrane ductal scaffolds nested within each other.

4. The method of claim 1, wherein the at least one chassis is configured so that microfluidic channels leading to the internal and the external compartments of the ductal scaffold are interconnected at one or more areas of the porous ductal scaffold locations.

5. The method of claim 1, wherein the features of the at least one chassis forming the microfluidic channels leading to the internal compartment of the ductal scaffold could extend beyond the inlet and outlet holes of the channel and later be plugged post-assembly of the chip sub-components.

6. The method of claim 1, wherein the cylindrical duct is formed by bonding the membranes and the at least one chassis by one of or a combination of chemical bonding, pressure bonding, or heat bonding.

7. The method of claim 1, wherein the curved membranes forming the cylindrical ductal scaffold can be bonded prior or post to assembly in the at least one chassis.

8. The method of claim 1, wherein the bonding method is used to melt a controlled thickness of the materials surfaces, welding the different parts together.

9. The method of claim 1, wherein the chemical bonding contains a mixture of ethanol and chloroform.

10. The method of claim 1, wherein the at least one coverslip glass forming the top and bottom layers of the biochip is bonded to the at least one chassis using the same method as the other parts or using a glass-polymeric glue.

11. The method of claim 1, wherein the plugs closing the extremities of the microfluidic channels leading to the ductal scaffold inner compartment, are bonded or using a polymeric glue.

12. The method of claim 1, wherein the extension of the ductal scaffold membrane are held at its extremities and tensioned to prevent any wrinkling in the membranes.

13. The method of claim 1, wherein a round porous duct is formed by curving a flat membrane over a rod in a closed loop of cylindrical cross-section and bonding it in an area on the ductal scaffold surface along its length.

14. The method of claim 1, wherein a round porous duct is formed by curving a flat membrane over a rod in a closed loop of cylindrical cross-section and bonding it in a flat area along the surface of the ductal scaffold that is an extension of the duct surface.

15. The method of claim 1, wherein a round porous duct is formed by curving more than one flat membrane over a rod in a closed loop of cylindrical cross-section and bonding it in a flat area along the surface of the ductal scaffold that is extension of the duct surface.

16. The method of claim 1, wherein forming the membranes includes a rod holding the membrane into its desired shapes is located in between the at least one chassis, with the extremities of the ductal scaffold is bonded to the chassis and surrounded by the at least one chassis.

## Patentansprüche

1. Verfahren zum Herstellen eines Biochips, das Verfahren umfassend:
Bereitstellen mindestens eines Chassis, wobei das mindestens eine Chassis einschließt,
einen Kanal, der konfiguriert ist, um die mindestens eine Membranstruktur zu tragen, und
mindestens einen Mikrofluidkanal in Fluidkommunikation mit dem Kanal;
Bereitstellen mindestens einer porösen Membran;
Krümmen mindestens einer porösen Membran in eine geschlossene Schleife mit zylindrischem Querschnitt,
wobei ein rundes, zylindrisches poröses Rohr durch Krümmen eines ersten Teils einer porösen Membran ausgebildet wird und eine oder mehrere zusätzliche poröse Membranen einen zweiten Teil des Rohrs ausbilden, und
Einfügen der mindestens einen porösen Membran zwischen das mindestens eine Chassis.

2. Verfahren nach Anspruch 1, wobei das mindestens eine Chassis konfiguriert ist, um Merkmale zu enthalten, die die Mikrofluidkanäle ausbilden, die zu den inneren und den äußeren Kompartimenten des Rohrgerüsts führen.

3. Verfahren nach Anspruch 1, wobei mindestens eine Membranstruktur innerhalb des inneren Raums innerhalb der zylindrischen Gerüste der mindestens einen Membranstruktur kombiniert wird, wobei Schichten ineinander verschachtelter poröser Membranrohrgerüste bereitgestellt werden.

4. Verfahren nach Anspruch 1, wobei das mindestens eine Chassis konfiguriert ist, sodass Mikrofluidkanäle, die zu den inneren und den äußeren Kompartimenten des Rohrgerüsts führen, an einem oder mehreren Bereichen der Stellen des porösen Rohrgerüsts miteinander verbunden sind.

5. Verfahren nach Anspruch 1, wobei die Merkmale des mindestens einen Chassis, die die Mikrofluidkanäle ausbilden, die zu dem inneren Kompartiment des Rohrgerüsts führen, sich über die Einlass- und Auslasslöcher des Kanals hinaus erstrecken könnten und später nach einer Montage der Chip-Unterkomponenten verschlossen werden könnten.

6. Verfahren nach Anspruch 1, wobei das zylindrische Rohr durch Binden der Membranen und des mindestens einen Chassis durch eine von chemischer Bindung, Druckbindung oder Wärmebindung oder eine Kombination davon ausgebildet wird.

7. Verfahren nach Anspruch 1, wobei die gekrümmten Membranen, die das zylindrische Rohrgerüst ausbilden, vor oder nach der Montage in dem mindestens einen Chassis gebunden werden können.

8. Verfahren nach Anspruch 1, wobei das Bindeverfahren verwendet wird, um eine kontrollierte Dicke der Materialoberflächen zu schmelzen, wobei die verschiedenen Teile miteinander verschweißt werden.

9. Verfahren nach Anspruch 1, wobei die chemische Bindung eine Mischung aus Ethanol und Chloroform enthält.

10. Verfahren nach Anspruch 1, wobei das mindestens eine Deckglas, das die obere und untere Schicht des Biochips ausbildet, mit dem mindestens einen Chassis unter Verwendung des gleichen Verfahrens wie die anderen Teile oder unter Verwendung eines Glas-Polymer-Klebers verbunden wird.

11. Verfahren nach Anspruch 1, wobei die Verschlüsse, die die Enden der Mikrofluidkanäle verschließen, die zu dem inneren Kompartiment des Rohrgerüsts führen, miteinander gebunden werden oder ein Polymerkleber verwendet wird.

12. Verfahren nach Anspruch 1, wobei die Verlängerung der Rohrgerüstmembran an ihren Enden festgehalten und gespannt werden, um eine etwaige Faltenbildung in den Membranen zu verhindern.

13. Verfahren nach Anspruch 1, wobei ein rundes poröses Rohr durch Krümmen einer flachen Membran über einem Stab in einer geschlossenen Schleife mit zylindrischem Querschnitt und durch Binden dieser in einem Bereich auf der Rohrgerüstoberfläche entlang ihrer Länge ausgebildet wird.

14. Verfahren nach Anspruch 1, wobei ein rundes poröses Rohr durch Krümmen einer flachen Membran über einem Stab in einer geschlossenen Schleife mit zylindrischem Querschnitt und durch Binden dieser in einem flachen Bereich entlang der Oberfläche des Rohrgerüsts ausgebildet wird, was eine Verlängerung der Rohroberfläche ist.

15. Verfahren nach Anspruch 1, wobei ein rundes poröses Rohr durch Krümmen mehr als einer flachen Membran über einem Stab in einer geschlossenen Schleife mit zylindrischem Querschnitt und durch Binden dieser in einem Bereich entlang der Oberfläche des Rohrgerüsts ausgebildet wird, was die Verlängerung der Rohroberfläche ist.

16. Verfahren nach Anspruch 1, wobei das Ausbilden der Membranen einen Stab einschließt, der sich zwischen dem mindestens einen Chassis befindet, der die Membran in der gewünschten Form hält, wobei die Enden des Rohrgerüsts mit dem Chassis gebunden und durch das mindestens eine Chassis umgeben sind.

## Revendications

1. Procédé de fabrication d'une biopuce, le procédé comprenant :
la fourniture d'au moins un châssis, dans lequel l'au moins un châssis comporte,
un canal conçu pour supporter l'au moins une structure membranaire et
au moins un canal microfluidique en communication fluidique avec le canal ;
la fourniture d'au moins une membrane poreuse ;
le fait de courber au moins une membrane poreuse en une boucle fermée de section transversale cylindrique,
dans lequel un conduit poreux, rond et cylindrique est formé par le fait de courber une première partie d'une membrane poreuse et une ou plusieurs membranes poreuses supplémentaires forment une seconde partie du conduit, et
l'insertion de l'au moins une membrane poreuse entre l'au moins un châssis.

2. Procédé selon la revendication 1, dans lequel l'au moins un châssis est conçu pour contenir des éléments formant les canaux microfluidiques menant aux compartiments interne et externe de l'échafaudage de conduit.

3. Procédé selon la revendication 1, dans lequel au moins une structure membranaire est combinée dans l'espace interne dans les échafaudages cylindriques de l'au moins une structure membranaire, fournissant des couches d'échafaudages de conduit de membrane poreuse imbriquées les unes dans les autres.

4. Procédé selon la revendication 1, dans lequel l'au moins un châssis est conçu de sorte que les canaux microfluidiques menant aux compartiments interne et externe de l'échafaudage de conduit sont reliés entre eux au niveau d'une ou de plusieurs zones des emplacements d'échafaudage de conduit poreux.

5. Procédé selon la revendication 1, dans lequel les éléments de l'au moins un châssis formant les canaux microfluidiques menant au compartiment interne de l'échafaudage de conduit pourraient s'étendre au-delà des trous d'entrée et de sortie du canal et être ultérieurement bouchés après l'assemblage des sous-composants de la puce.

6. Procédé selon la revendication 1, dans lequel le conduit cylindrique est formé par la liaison des membranes et de l'au moins un châssis par l'une parmi liaison chimique, liaison par pression ou liaison par chaleur, ou une combinaison de celles-ci.

7. Procédé selon la revendication 1, dans lequel les membranes courbées formant l'échafaudage de conduit cylindrique peuvent être reliées avant ou après l'assemblage dans l'au moins un châssis.

8. Procédé selon la revendication 1, dans lequel le procédé de liaison est utilisé pour faire fondre une épaisseur contrôlée des surfaces des matériaux, ce qui soude les différentes parties ensemble.

9. Procédé selon la revendication 1, dans lequel la liaison chimique contient un mélange d'éthanol et de chloroforme.

10. Procédé selon la revendication 1, dans lequel l'au moins une lamelle couvre-objet en verre formant les couches supérieure et inférieure de la biopuce est reliée à l'au moins un châssis en utilisant le même procédé que pour les autres parties ou en utilisant une colle verre-polymère.

11. Procédé selon la revendication 1, dans lequel les bouchons fermant les extrémités des canaux microfluidiques menant au compartiment interne d'échafaudage de conduit sont reliés ou utilisent une colle polymère.

12. Procédé selon la revendication 1, dans lequel les extensions de la membrane d'échafaudage de conduit sont maintenues au niveau de leurs extrémités et tendues pour éviter tout plissement dans les membranes.

13. Procédé selon la revendication 1, dans lequel un conduit poreux rond est formé en courbant une membrane plate sur une tige en une boucle fermée de section transversale cylindrique et en la reliant dans une zone sur la surface d'échafaudage de conduit le long de sa longueur.

14. Procédé selon la revendication 1, dans lequel un conduit poreux rond est formé en courbant une membrane plate sur une tige en une boucle fermée de section transversale cylindrique et en la reliant dans une zone plate le long de la surface de l'échafaudage de conduit qui est une extension de la surface de conduit.

15. Procédé selon la revendication 1, dans lequel un conduit poreux rond est formé en courbant plus d'une membrane plate sur une tige en une boucle fermée de section transversale cylindrique et en la reliant dans une zone plate le long de la surface de l'échafaudage de conduit qui est une extension de la surface de conduit.

16. Procédé selon la revendication 1, dans lequel le formage des membranes comporte une tige maintenant la membrane dans ses formes souhaitées est située entre l'au moins un châssis, avec les extrémités de l'échafaudage de conduit est reliée au châssis et entourée par l'au moins un châssis.
